(19) Europäisches Patentamt
European Patent Office
Office européen des brevets

(11) EP 2 497 574 A1

(12) EUROPEAN PATENT APPLICATION
published in accordance with Art. 153(4) EPC

(43) Date of publication:
**12.09.2012 Bulletin 2012/37**

(21) Application number: **10828377.1**

(22) Date of filing: **08.11.2010**

(51) Int Cl.:
***B01J 31/10*** *(2006.01)* ***C07C 37/20*** *(2006.01)*
***C07C 39/16*** *(2006.01)* ***C07D 213/32*** *(2006.01)*
***C07B 61/00*** *(2006.01)*

(86) International application number:
**PCT/JP2010/069835**

(87) International publication number:
**WO 2011/055819 (12.05.2011 Gazette 2011/19)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.11.2009 JP 2009255109**

(71) Applicant: **Mitsubishi Chemical Corporation**
**Chiyoda-ku**
**Tokyo 100-8251 (JP)**

(72) Inventors:
- **TSUTSUMINAI, Susumu**
  **Ibaraki 314-0102 (JP)**
- **ANDO, Shingo**
  **Fukuoka 806004 (JP)**

(74) Representative: **Jappy, John William Graham**
**Gill Jennings & Every LLP**
**The Broadgate Tower**
**20 Primrose Street**
**London EC2A 2ES (GB)**

# (54) CATALYST FOR PRODUCTION OF BISPHENOL COMPOUND AND METHOD FOR PRODUCING BISPHENOL COMPOUND

(57) A catalyst for producing a bisphenol compound, composed of gel catalyst beads formed by introducing strongly acidic group such as sulfonic acid group into gel beads obtained by the copolymerization of a styrene-based monomer and a crosslinking monomer, wherein 50% or more of the gel catalyst beads have particle diameters of 30 to 650 μm, and a method for producing a bisphenol compound by the reaction of a phenol compound and a carbonyl compound in the presence of the catalyst. The present invention provides a strongly acidic cation exchange resin catalyst for producing a bisphenol compound, exhibiting high raw-material conversion rate and high bisphenol-compound selectivity, having a long catalyst life span, and reducing pressure loss in a catalyst filled layer, and a method for producing a bisphenol compound at high conversion rate and high selectivity stably and efficiently for a long time by using the catalyst.

EP 2 497 574 A1

## Description

### TECHNICAL FIELD

The present invention relates to a catalyst for producing a bisphenol compound and a method for producing a bisphenol compound. More particularly, the invention relates to a strongly acidic cation exchange resin catalyst for producing a bisphenol compound, which catalyst has high activity and high selectivity, and a method for producing a bisphenol compound using the catalyst, which method stably and efficiently produces a bisphenol compound at high conversion rate and high selectivity for a long period of time.

### BACKGROUND ART

In general, a bisphenol compound is produced by the condensation reaction of an aromatic hydroxy compound (a phenol compound) and a carbonyl compound in the presence of an acidic catalyst. As acidic catalysts, mineral acids such as hydrochloric acid and sulfuric acid, solid acids such as heteropoly acid, and the like, are used. However, in terms of apparatus corrosion due to catalyst and reaction activity, catalyst cost, and the like, cation exchange resins having an acidic group such as sulfonic acid are widely used in industry. In addition, to improve conversion rate, selectivity, and the like, there is known a method in which reaction is conducted by mixing a compound containing a mercapto group or a protected mercapto group, (which hereinafter may be referred to as "mercapto compound") together with a catalyst.

As methods for mixing a mercapto compound together with a catalyst, there are known methods, such as sequential feeding of a mercapto compound with a phenol compound and a carbonyl compound as raw materials into a catalyst to conduct reaction (for example, see Patent Document 1 and 2), and using a mercapto compound having a functional group capable of bonding to a sulfonic acid group such as an amino group (for example, mercaptoalkyl amines and mercaptoalkyl pyridines) to modify a sulfonic acid group of a sulfonic acid cation exchange resin prior to reaction (for example, see Patent Document 3).

In addition, various improvements have been made in sulfonic acid cation exchange resins as acidic catalyst. One example of that is Patent Document 4 showing that using a sulfonic acid cation exchange resin in a microparticle- and/or powder form having an effective diameter of 0.3 mm or less greatly improves catalytic activity, and particularly, macroporous resin is significantly effective to improve the activity. The Document has reported an example of producing bisphenol A using a resin with a mean particle diameter of 30 to 60 $\mu$m obtained by crushing a macroporous sulfonic acid cation exchange resin.

Additionally, Patent Document 5 has described that, by producing a sulfonic acid cation exchange resin catalyst from copolymer beads obtained by ejecting a mixture of a styrene-based monomer, a crosslinking agent monomer, and a polymerization initiator into an aqueous liquid to produce liquid droplets and conducting suspension polymerization, a conversion rate of the reaction product to a bisphenol becomes higher than in a catalyst produced without ejection, and has reported an example of producing bisphenol A using a sulfonic acid cation exchange resin having particle diameters of 425 to 600 $\mu$m produced by the ejection of liquid droplets. The Document, however, has not examined the relationship between the particle diameter of the sulfonic acid cation exchange resin and uniformity of the particle diameter, and 4,4'-bisphenol A selectivity of produced bisphenol A.

Patent Document 6 has proved that, by using, as the catalyst, a sulfonic acid cation exchange resin having a mean particle diameter of 0.2 to 2.0 mm and a uniform coefficient of 1.0 to 1.4, the pressure loss can be kept at low level even when a raw material mixture solution is fed into a catalyst filled layer at high liquid hourly space velocity, and has exemplified a result of evaluation and comparison of the pressure loss and catalyst capabilities of respective examples by sieving a sulfonic acid cation exchange resin having a mean particle diameter of 0.54 mm and a uniformity coefficient of 1.46 and extracting resin particles having a mean particle diameter of 0.69 mm and a uniform coefficient of 1.17. However, the Document has not suggested any improvement in acetone conversion rate and 4, 4'-bisphenol A selectivity, so that further improvement is needed in the catalytic activity and the selectivity.

To industrially produce a bisphenol compound, it is important to improve raw material conversion rate in reaction, the selectivity of an intended bisphenol compound, and a catalyst life span. Further, it is important to reduce pressure loss in a catalyst filled layer to low level to continue operation stably for a long period oftime. However, none ofthe above conventional techniques have yet sufficiently met requirements at industrial level and there has been a demand for catalysts having higher capabilities in industrial production.

### PRIOR ART REFERENCES

[Patent Document]

[Patent Document 1]: JP-A-2001-503377
[Patent Document 2]: JP-A-2002-205966
[Patent Document 3]: JP-A-2002-69023
[Patent Document 4]: JP-A-1987-178532
[Patent Document 5]: Japanese Patent No. 3312920
[Patent Document 6]: Japanese Patent No. 2887304

SUMMARY OF THE INVENTION

The present invention has been accomplished in view of the conventional circumstances as above. It is an object ofthe present invention to provide a strongly acidic cation exchange resin catalyst for producing a bisphenol compound, which catalyst has high raw material conversion rate and high selectivity of an intended bisphenol compound, has a long catalyst life span, and reduces pressure loss in a catalyst filled layer so that the intended bisphenol compound can be advantageously industrially produced, and a method for stably and efficiently producing an intended bisphenol compound at high conversion rate and high selectivity for a long period of time by using the strongly acidic cation exchange resin catalyst for producing a bisphenol compound.

To solve the above problems, the inventors of the present invention produced a catalyst for producing a bisphenol compound, which catalyst is composed of strongly acidic cation exchange resin catalyst beads (hereinafter may be referred to as "catalyst beads") having a structure in which sulfonic acid group has been introduced into a spherical copolymer obtained by the copolymerization of polymerizable monomers including a styrene-based monomer and a cross-linking monomer, in which 50% or more of the catalyst beads have particle diameters of 30 to 650 μm The inventors used the produced the catalyst in production reaction of a bisphenol compound and found that the catalyst can achieve improvement in selectivity, yield, and catalyst life span that were not sufficed by the conventional catalysts and can reduce the pressure loss in the catalyst filled layer to low level, thereby completed the present invention.

Specifically, the catalyst for producing a bisphenol compound according to the present invention (claim 1) is gel catalyst beads composed of gel beads obtained by the copolymerization reaction of polymerizable monomers including a styrene-based monomer and a crosslinking monomer and strongly acidic group introduced into the gel beads, wherein 50% or more ofthe gel catalyst beads have particle diameters of 30 to 650 μm.
The catalyst for producing a bisphenol compound according to claim 2 is characterized in that, when the catalyst according to clam 1 is used in a fixed-bed flow method, 50% or more of the gel catalyst beads have particle diameters of 300 to 600 μm
The catalyst for producing a bisphenol compound according to claim 3 is characterized in that, in the catalyst according to claim 1 or 2, a part of the strongly acidic group of the catalyst is modified with mercaptopyridine or mercaptoalkylpyridine having a protected mercapto group.
The catalyst for producing a bisphenol compound according to claim 4 is characterized in that, in the catalyst according to claim 3, the mercaptoalkylpyridine is 4-(2-mercaptoethyl)pyridine or 2-(2-mercaptoethyl)pyridine.
The method for producing a bisphenol compound according to claim 5 is characterized in that a phenol compound is reacted with a carbonyl compound in the presence ofthe catalyst for producing a bisphenol compound according to the present invention as described above.

The catalyst for producing a bisphenol compound according to the present invention has high raw material conversion rate and bisphenol compound selectivity, has a long catalyst life span, and can reduce pressure loss in a catalyst filled later to low level. Accordingly, by using the catalyst for producing a bisphenol compound ofthe present invention, the pressure loss in the catalyst filled layer is significantly suppressed and then high selectivity and yield are maintained, thereby a bisphenol compound can be produced stably and efficiently for a long period oftime.

BRIEF DESCRIPTION OF THE DRAWINGS

Fig. 1 is a schematic cross-sectional view showing a liquid droplet production apparatus and a polymerization reaction apparatus used to produce copolymers in Examples.
Fig. 2 is a plan view showing a nozzle member attached to the liquid droplet production apparatus of Fig. 1.
Fig. 3 is a system diagram showing an apparatus used to measure pressure loss of strongly acidic cation exchange resins in the Examples.
Fig. 4 is a graph showing time-dependent changes in acetone conversion rate in the production of bisphenol A of Example 18 and Comparative Example 4.
Fig. 5 is a graph showing time-dependent changes in 4,4'-bisphenol A selectivity in the production ofbisphenol A of Example 18 and Comparative Example 4.
Fig. 6 is a graph showing time-dependent changes in acetone conversion rate in the production ofbisphenol A of

Example 19 and Comparative Example 5.

Fig. 7 is a graph showing time-dependent changes in 4,4'-bisphenol A selectivity in the production ofbisphenol A of Example 19 and Comparative Example 5.

MODES FOR CARRYING OUT THE INVENTION

Hereinafter, explanation will be given in more detail for the embodiments of a catalyst for producing a bisphenol compound according to the present invention and a method for producing a bisphenol compound according to the present invention.

(1) Catalyst for Producing Bisphenol Compound

The catalyst for producing a bisphenol compound of the present invention is composed of gel catalyst beads comprising gel beads obtained by the copolymerization reaction of polymerizable monomers including a styrene-based monomer and a crosslinking monomer and strongly acidic group introduced into the gel beads, and in the catalyst, a ratio of gel catalyst beads having particle diameters of 30 to 650 μm accounts for 50% or more of the total.

The styrene-based monomer in the present invention refers to styrene or a monomer having an arbitrary substituent in a benzene ring or vinyl group of styrene in a range maintaining a function as an ion exchange resin. The styrene-based monomer may be a polymer such as polyester, polycarbonate, polyamide, polyolefin, poly(meth)acrylate ester, polyether, and polystyrene, and a macromonomer having a styryl structure at an end of oligomer. As used herein, the term "(meth)acryl" means "acryl" and "methacryl", and the same rule is applied to the term "(meth)acryloyl" described below.

The styrene-based monomer is preferably a monomer represented by the following formula (1):

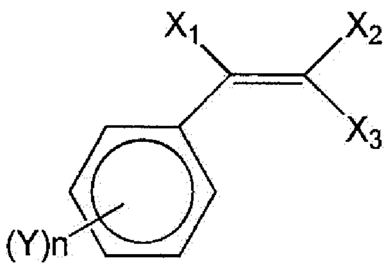

(1)

(In the formula, $X_1$, $X_2$, and $X_3$ represent any of a hydrogen atom, an alkyl group, an aryl group, an alkoxy group, a halogen atom, an alkylsilyloxy group, a nitro group, and a nitrile group; Y represents a hydrogen atom, an amino group, an alkylamino group, an alkyl group, an alkenyl group, an alkynyl group, a halogen atom, a haloalkyl group, aryl groups such as a phenyl group and an naphthyl group, aralkyl groups such as benzyl groups, alkoxyalkyl groups, nitro groups, alkanoyl groups, aroyl groups such as benzoyl groups, alkoxycarbonyl groups, arylalkoxycarbonyl groups, alkoxy groups, haloalkoxy groups, allyloxy groups, aralkyloxy groups, alkoxyalkyloxy groups, alkanoyloxy groups, alkoxycarbonyloxy groups, aralkyloxy carbonyloxy groups, or alkylsilyloxy groups; n represents an integer of 1 to 5; $X_1$, $X_2$, and $X_3$ may be the same or different from each other, in which when n is 2 or larger, a plurality of Ys may be the same or different).

Specific examples of the styrene-based monomer include styrene or styrenes in which a hydrogen atom of a benzene ring has been substituted with an alkyl group having 1 to 4 carbon atoms or a halogen atom, such as o-methylstyrene, m-methylstyrene, p-methylstyrene, o-ethylstyrene, m-ethylstyrene, p-ethylstyrene, fluorostyrene, chlorostyrene, and bromostyrene, and styrenes in which a hydrogen atom of a vinyl group has been substituted with an alkyl group having 1 to 4 carbon atoms or a halogen atom, such as α-methylstyrene, α-fluorostyrene, and β-fluorostyrene. Among them, the most preferable styrene-based monomer is styrene. In addition, those styrene-based monomers may be used alone or in combinations of two or more thereof

The crosslinking monomer is a compound having two or more carbon-carbon double bonds capable of being copolymerized with any of the styrene-based monomers in the molecule. Specific examples of the crosslinking monomer include polyvinyl benzenes such as divinylbenzene and trivinylbenzene, alkyl divinylbenzenes such as divinyltoluene, and aromatic divinyl compounds having a structure in which two or more benzene rings have been bonded together directly or via a linking group such as an alkyl group or a styrylene group, which may be bis(vinylphenyl), bis(vinylphenyl) methane, bis(vinylphenyl)ethane, bis(vinylphenyl)propane, and bis(4-vinylphenyl)sulfone. In addition, the crosslinking compounds may be polymers such as polyester, polycarbonate, polyamide, polyolefin, polymethacrylate, polyether, or polystyrene, and macromonomers having a polymerizable carbon-carbon double bond such as a styryl structure or

(meth)acryl structure at both ends of oligomer. Among them, preferable crosslinking monomers are divinylbenzenes. Depending on the divinylbenzene, when it is produced, ethylvinylbenzene (ethylstyrene) may be produced as a by-product and contained in large amount. The present invention can also use such a divinylbenzene.
Those crosslinking monomers may be used alone or in combinations oftwo or more thereof

The polymerizable monomers in the present invention include a styrene-based monomer and a crosslinking monomer. Other than those, as needed, the polymerizable monomers may further include any other monomer that is polymerizable with them. Specific examples of such a polymerizable monomer (hereinafter may be referred to as "third monomer") include vinyl monomers having polycyclic aromatic skeletons such as such as naphthalene, anthracene, and phenanthrene, for example, vinylnaphthalene and vinylanthracene; phenanthrenes; (meth)acrylate esters such as methyl acrylate, ethyl acrylate, methyl methacrylate, and ethyl methacrylate, diene-based hydrocarbon compounds such as butadienes and isoprenes; α-olefins such as 1-pentene and 1-hexene; and (meth)acrylonitrile. These compounds may be used alone or in combinations of two or more thereof.

By using such a third monomer, there can be obtained advantages, such as increased acid resistance. In this case, the amount of the third monomer to be used is usually 50% by mole or less with respect to the styrene-based monomer, preferably 20% by mole or less with respect thereto, and particularly preferably 10% by mole or less with respect thereto. When the amount of the third monomer used is too much, the amount of strongly acidic group per unit weight that can be introduced into an obtained copolymer is reduced, so that an intended catalytic activity may not be obtainable.

The degree of crosslinking in the gel beads as the copolymer obtained by the polymerization of polymerizable monomers including a styrene-based monomer and a crosslinking monomer is preferably 1 % or more, and more preferably 2% or more, and preferably 8% or less, and more preferably 5% or less. The degree of crosslinking as used herein refers to a concentration on a weight basis of the crosslinking monomer in polymerizable monomers to be polymerized. The meaning is the same as the definition used in the relevant field
When the crosslinking degree is too small, it is difficult to maintain the strength of the obtained catalyst for producing a bisphenol compound. Thereby, when used as a bisphenol compound-producing catalyst in reaction, fracture or the like occurs in the catalyst beads due to swelling and contraction occurring when conditioning is performed by contacting it with a phenol compound, a mixture solvent of the phenol compound and water, or the like before its use, which is unfavorable. Meanwhile, an excessively large degree of crosslinking makes it difficult to cause swelling of the obtained catalyst beads, whereby diffusive resistance in the catalyst beads easily occurs, which significantly reduces catalytic activity unfavorably.

The copolymerization reaction of polymerizable monomers including a styrene-based monomer and a crosslinking monomer can be conducted based on a known technique using a radical polymerization initiator. Examples of the radical polymerization initiator to be used include one of or two or more of benzoyl peroxides, lauroyl peroxide, t-butyl hydroperoxide, and azobisisobutyronitrile. The radical polymerization initiator is usually in an amount of 0.05% by weight or more and 5% by weight or less with respect to the weight of polymerizable monomers (a total monomer weight).

Polymerization form is not specifically restricted and polymerization can be conducted in a variety of forms, such as solution polymerization, emulsification polymerization, or suspension polymerization. To set a uniformity coefficient and a mean particle diameter described below in specified ranges, sieve classification or the like may also be conducted. The present invention suitably uses a known method for obtaining a spherical copolymer having a uniform particle diameter. For example, a method is known in which, before polymerization, an oil-in-water type dispersion solution in which monomer-containing liquid droplets with a uniform particle diameter have been dispersed, is produced and fed into a polymerization container to conduct polymerization. As a method for producing the oil in-water type dispersion solution with a uniform particle diameter, there can be used a method in which there is disposed a nozzle plate with ejection holes formed upward at a bottom portion of a container filled with water; and through the ejection holes, a monomer-containing liquid is supplied into the water to disperse monomer-containing liquid droplets in the water (for example, see JP-A-2003-252908, and Japanese Patent No. 3899786). In the Examples described below, this method is employed.

The mean particle diameter and the uniformity coefficient of the gel beads can be calculated from a particle diameter distribution measured by a sieving method shown in "Manual 1 of DIAION: Ion Exchange Resign. Synthetic Absorbent" (published by Mitsubishi Chemical Corp., 4th Revised Edition, issued on Oct. 31, 2007, pp.140-141).
Polymerization temperature in the copolymerization reaction is usually room temperature (approximately 18 to 25°C) or higher, preferably 40°C or higher, and more preferably 70°C or higher, and usually 250°C or lower, preferably 150°C or lower, and more preferably 140°C or lower. When the polymerization temperature is too high, depolymerization concurs and thereby the degree of polymerization completion rather decreases. When the polymerization temperature is too low, the degree of polymerization completion becomes insufficient. In addition, polymerization can be conducted under an atmosphere of air or inert gas, and the inert gas to be used can be, for example, nitrogen, carbon dioxide, or argon.

The method for introducing strongly acidic group into the gel beads as the copolymer obtained by the above-described copolymerization reaction is not specifically restricted and the introduction can be done according to a usual

method. The strongly acidic group is preferably a sulfonic acid group. The method for introducing a sulfonic acid group (sulfonation) is conducted, for example, in the absence of any organic solvent or in the presence of an organic solvent such as benzene, toluene, xylene, nitrobenzene, chlorobenzene, tetrachloromethane, dichloroethane, trichloroethylene, or propylene dichloride, by the reaction of the gel beads as the copolymer with a sulfonating agent such as sulfuric acid, chlorosulfonate, or fuming sulfuric acid. As used herein, as each ofthe organic solvent and the sulfonating agent, one compound may be used alone, or two or more compounds may be used in combination. In this case, reaction temperature is usually approximately 0 to 150°C and appropriately selected according to the sulfonating agent and the organic solvent to be used.

Gel beads into which strongly acidic group has been introduced are separated by washing and isolating or the like according to a usual method to obtain gel catalyst beads as a strongly acidic cation exchange resin.

In the present invention, an exchange capacity (an amount of strongly acidic group such as sulfonic acid group) as the gel catalyst beads is usually 0.5 meq/mL or more per unit volume of resin containing water, and preferably 1.0 meq/mL or more per unit volume thereof, and is usually 3.0 meq/mL or less and preferably 2.0 meq/mL or less per unit volume thereof Furthermore, in a dried resin, the exchange capacity is usually 1.0 meq/g or more per unit volume of the resin, and preferably 2.0 meq/g or more per unit volume thereof and is usually 6.0 meq/g or less and preferably 5.5 meq/g or less per unit volume thereof In a wet state in which adherent water has been removed from the water-containing resin, the exchange capacity is usually 0.5 meq/g or more and preferably 1.0 meq/g or more per unit volume of the resin, and is usually 3.0 meq/g or less and preferably 2.0 meq/g or less per unit volume thereof When the exchange capacity is too low, catalytic activity becomes insufficient, whereas a cation exchange resin having an excessively high exchange capacity cannot be produced easily.

The exchange capacity of the gel catalyst beads as the strongly acidic cation exchange resin can be obtained by a method shown in, for example, "Manual 1 of DIAION: Ion Exchange Resign. Synthetic Absorbent" (published by Mitsubishi Chemical Corp., 4th Revised Edition, issued on Oct. 31, 2007, pp. 133-135) or a method based on the manual.

The catalyst for producing a bisphenol compound according to the present invention is composed of gel catalyst beads, among which gel catalyst beads having particle diameters of 30 to 650 μm accounts for 50% or more, preferably 60% or more, more preferably 80% or more, and most preferably 90% or more of the total.

When using the catalyst for producing a bisphenol compound in a fixed-bed flow method, the catalyst preferably contains gel catalyst beads, among which gel catalyst beads having particle diameters of 300 to 600 μm accounts for 50% or more.

When 50% or more of all the gel catalyst beads forming the catalyst for producing a bisphenol compound according to the present invention have the particle diameters of 30 to 650 μm, there can be obtained excellent catalyst capabilities in terms of catalytic activity and the selectivity of an intended bisphenol compound Meanwhile, if those having the particle diameters of 30 to 650 μm account for less than 50% of the total, the catalytic activity is reduced due to diffusive resistance in the catalyst particles and also successive reaction in the catalyst particles causes the reduction of the selectivity.

If the mean particle diameter of the gel catalyst beads forming the catalyst for producing a bisphenol compound according to the present invention is less than 100 μm, it is necessary to significantly increase a pressure for supplying a raw material to a catalyst layer. This increases the magnitude of force applied to the catalyst particles, which easily causes abrasion and miniaturization of the catalyst particles, resulting in shortening of the life span of the catalyst filled layer. In addition, as the raw-material supplying pressure becomes higher, the energy consumption rate becomes larger, and thus economical efficiency in the process becomes poor, so that the mean particle diameter is preferably 100 μm or more. More preferably, the mean particle diameter is 300 μm or more, since pressure loss in the catalyst filled layer can be reduced to low level when used in the fixed-bed flow method.

Furthermore, when the uniformity coefficient of particle diameter ofthe gel catalyst beads forming the catalyst for producing a bisphenol compound ofthe present invention is 1.40 or less, the pressure loss in the catalyst filled layer can be reduced to low level when used in the fixed-bed flow method. Therefore, in the case of using the catalyst in a fixed bed, the uniformity coefficient is preferably 1.10 or less, since the same advantageous effect as above becomes further improved. On the other hand, if the uniformity coefficient is larger than 1.40, it is necessary to significantly increase the raw-material supplying pressure to the catalyst layer, and force applied to the catalyst particles becomes larger, which easily causes abrasion and miniaturization of the catalyst particles. This shortens the life span ofthe catalyst filled layer. In addition, the higher the raw material supply pressure, the larger the energy consumption rate, so that the economical efficiency in the process becomes poor, which is undesirable.

Regarding the gel catalyst beads in the present specification, the mean particle diameter and the uniformity coefficient are values calculated from the particle diameter distribution measured by a sieving method shown in "Manual 1 of DIAION: Ion Exchange Resin · Synthetic Absorbent" (published by Mitsubishi Chemical Corp., 4th Revised Edition, issued on Oct. 31, 2007, pp.140-141) using the following formulas.

Mean particle diameter = diameter equivalent to 50% of cumulative volume of resin.

Uniformity coefficient = diameter in which cumulative volume of larger particles is equivalent to 40% / diameter in which cumulative volume of larger particles is equivalent to 90%.

Alternatively, by calculation of measured values obtained using a method such as centrifugal sedimentation, Coulter method, image analysis method, or laser diffraction scattering, other than sieving, the calculated values can also be used as the values of the sieving method.

In the gel catalyst beads of the present invention, preferably, a part of the strongly acidic group is modified with a compound containing a mercapto group or a protected mercapto group. In this case, the method for modifying the strongly acidic group of the gel catalyst beads is not specifically restricted. As a typical method therefor, there can be mentioned a method in which a mercapto compound containing a mercapto group or a protected mercapto group and also containing a functional group, such as an amino group or a pyridine ring capable of forming an ionic bond with the strongly acidic group of the gel catalyst beads is reacted with the gel catalyst beads in an aqueous solvent or an organic solvent to form an ionic bond with a strongly acidic group such as a sulfonic acid group of a cation exchange resin. Specific examples of the method include a method in which a solution prepared by dissolving the mercapto compound in an appropriate solvent such as water, alcohol, ketone, ether, or phenol, or a mercapto compound not diluted with any solvent is directly mixed into the gel catalyst beads dispersed in a solvent by dropping or the like to stir the mixture. With the method, a part ofthe strongly acidic group of the gel catalyst beads reacts with the mercapto compound to form an ionic bond therewith so as to be neutralized and modified.

The mercapto compound to be used for modification is not specifically restricted as long as it is a compound forming an ionic bond with the strongly acidic group of the gel catalyst beads. Examples of such a mercapto compound include mercapto alkylamines such as 2-mercapto ethylamine, 3-mercapto propylamine, and N,N-dimetyl-3-mercapto propylamine; mercapto alkylpyridines such as 3-mercapto methylpyridine, 2-(2-mercaptoethyl)pyridine, 3-(2-mercaptoethyl)pyridine, and 4-(2-mercaptoethyl)pyridine; thiazolidines such as thiazolidine, 2,2-dimethyl thiazolidine, 2-methyl-2-phenyl thiazolidine, and 3-methyl thiazolidine, and derivatives thereof having a protected mercapto group. These compounds may be used alone or in combinations of two or more thereof

Additionally, preferably, these mercapto compounds are purified to be highly pure, but may include an impurity such as disulfide as long as it does not significantly inhibit reaction when modified gel catalyst beads are used as a catalyst. Futthermore, to prevent the generation of such an impurity, there may be used a stable salt ofthe mercapto compound obtained by reacting with a mineral acid such as hydrochloric acid or sulfuric acid.

Among them, as a promoter to be used in the production of a bisphenol compound, preferred are mercapto alkylpyridines, since they are very effective for the improvement in conversion rate and selectivity. Above all, preferred are 2-(2-mercaptoethyl)pyridine and 4-(2-mercaptoethyl)pyridine, since they are particularly greatly effective in that, when they are used to produce a bisphenol compound in the context of the catalyst for a bisphenol compound according to the present invention, the raw material conversion rate and the selectivity are improved and there is less activity reduction in the long-term use, as compared to other promoters. In addition, as a promoter, 2-(2-mercaptoethyl)pyridine is preferably used, since activity reduction is less in a long-term use in the context of the catalyst for a bisphenol compound according to the present invention, as compared to the use of 4-(2-mercaptoethyl)pyridine.

The protective group of the mercapto group is not specifically restricted as long as it can protect a mercapto group, and for example, the protection of mercapto group can be made using protective groups and a protection method described in "Green's Protective Groups in Organic Synthesis, Fourth Edition, Wiley (2007)". Specific examples include thioether derivatives protected with an aliphatic protective group that produces a stable carbocation, such as a tert-butyl group, thiocarbonate derivatives protected with a carbonate protective group, benzyl ether derivatives protected with a benzyl protective group, and dithioacetal derivatives protected with ketone or aldehyde.

The ratio in which the gel catalyst beads ofthe present invention are modified with a mercapto compound (modification rate) is preferably 3% by mole or more and more preferably 5% by mole of all strongly acidic groups ofthe gel catalyst beads, and also it is preferably 70% by mole or less, still preferably 50% by mole or less, and more preferably 30% or less thereof Thereby, without causing catalytic activity reduction due to reduction in the amount of strongly acidic groups necessary for condensation reaction in the production of a bisphenol compound, the effect of a mercapto compound acting as a promoter can be maximally exhibited. When the modification rate is too small, there tends to be less effective on the improvement of reactivity and thus the activity and life span of catalyst tend to become insufficient. Conversely, when the modification rate is too large, the amount of strongly acidic groups involved in reaction is reduced, so that the reactivity tends to be reduced. It is also economically unpreferable since a large amount of an expensive

mercapto compound is used.

(2) Method for Producing Bisphenol Compound

A method for producing a bisphenol compound according to the present invention is characterized in that a phenol compound is condensed with a carbonyl compound in the presence of the catalyst for producing a bisphenol compound of the present invention as described above.

It is considered that the condensation reaction of a phenol compound and a carbonyl compound uses a strong ortho-para orientation, particularly para orientation of phenolic hydroxyl group. Accordingly, preferably, a phenol compound to be used has no substituent at ortho position or para position. Particularly, a bisphenol compound as a condensation reaction product is, in general, preferably a 4,4'-bisphenol compound, from the viewpoint of its use. Thus, from this point, it is preferable to use a phenol compound having no substituent at para position

By using such a raw material, the property of having high selectivity of 4,4'-bisphenol compound in the catalyst for producing a bisphenol compound of the present invention can be sufficiently exhibited.

In the case of a phenol compound having a substituent, the substituent can be an arbitrary one according to the uses and physical properties of an obtained bisphenol compound, as long as the substituent does not inhibit the ortho-para orientation of a phenolic hydroxyl group and does not pose a steric hindrance to the condensation position of a carbonyl compound. As typical substituents, for example, there may be mentioned lower alkyl groups having 1 to 4 carbon atoms. In addition, instead of the substituent, among phenol compounds in which halogen atoms such as a fluorine atom, a chlorine atom, and a bromine atom are substituted, compounds having the same substitution position can also be used. The number of substituents can be one or more.

Specific examples of the phenol compound to be used in the present invention include phenol (unsubstituted phenol), o-cresol, m-cresol, 2,5-xylenol, 2,6-xylenol, 2,3,6-trimethylphenol, 2,6-di tert-butylphenol, o-chlorophenol, m-chlorophenol, 2,5-dichlorophenol, and 2,6-dichlorophenol. Among them, phenol is particularly preferred.

The carbonyl compound to be used in the present invention is not specifically restricted. Specific examples of the carbonyl compound include ketones having approximately 3 to 10 carbon atoms, such as acetone, methyl ethyl ketone, diethyl ketone, methyl isobutyl ketone, cyclohexanone, and acetophenone, and aldehydes having approximately 1 to 6 carbon atoms, such as formaldehyde, acetaldehyde, propionaldehyde, and butylaldehyde. Among them, acetone is preferable.

It is particularly preferable to use phenol as the phenol compound and acetone as the carbonyl compound, since there can be obtained bisphenol A useful as a raw material of polycarbonate resin or the like.

In the production method according to the present invention, to conduct the condensation reaction of a phenol compound and a carbonyl compound, it is possible to arbitrarily select a continuous method, semi-continuous method, batch method, and the like according to restrictions or the like to the amount of production and the apparatus. Only one reactor may be used or a plurality of reactors may be connected in parallel or in series for the production, or the production may be performed in combinations of those methods and the reactors. Those production methods may be used as an only reaction method. As another method, for example, there can be selected a method in which the continuous method and the batch method are performed in parallel using the plurality of reactors.

When reaction is conducted, the phenol compound and the carbonyl compound may be separately supplied into the reactor or may be mixed together to be supplied. Alternatively, when staring reaction, the carbonyl compound may be used in reaction all at one time or may be divided into portions to be added a plurality of time.

The ratio of an amount by mole of the phenol compound to an amount by mole of the carbonyl compound used in reaction ((amount by mole of phenol compound)/(amount by mole of carbonyl compound)) is usually in a range of 3 to 100, preferably in a range of 4 to 50, still preferably in a range of 5 to 25, and particularly preferably in a range of 6 to 20.

Alternatively, when conducting the reaction of a phenol compound and a carbonyl compound, a mercapto compound used for modification as described above and/or a mercapto compound that cannot be used for modification may exist in the reaction. As used herein, the term "mercapto compound that cannot be used for modification" refers to a mercapto compound that contains a mercapto group or a protected mercapto group but does not contain any functional group that can form an ionic bond with a strongly acidic group such as a sulfonic acid group of a cation exchange resin, and for example, there may be mentioned alkyl mercaptanes such as methyl mercaptanes and ethyl mercaptanes. These may be used alone or in combinations of a plurality of them. They may be used in reaction by dissolving in the phenol compound and/or the carbonyl compound or separately from the phenol compound and/or the carbonyl compound.

Additionally, as the reaction apparatus, there can be used a variety of apparatuses as needed, such as a reactor equipped with a heater and a cooler and an insulated reactor.

In the production method of the present invention, a preferable reaction method is a continuous method from the viewpoint of reaction efficiency and operational easiness. The continuous method is not specifically restricted as long as it is a method that conducts reaction by continuously feeding a phenol compound and a carbonyl compound in a reactor filled with the catalyst for producing a bisphenol compound of the present invention. For example, any of a

fixed-bed flow method, a flow bed method, and a continuous stirring method may be used. Particularly, preferred is the fixed-bed flow method in which the catalyst for producing a bisphenol compound is set as a fixed bed and a phenol compound and a carbonyl compound are continuously fed and circulated.

In the method for producing a bisphenol compound according to the present invention, when conducing the condensation reaction using the fixed-bed flow method, the flow bed method, and the continuous stirring method, the phenol compound and the carbonyl compound as raw materials are fed at a liquid hourly space velocity (LHSV) of usually 0.05 $hr^{-1}$ or more, and preferably 0.2 $hr^{-1}$ or more, and usually 20 $hr^{-1}$ or less and preferably 10 $hr^{-1}$ or less in a total of the phenol compound and the carbonyl compound, based on the gel catalyst beads in a wet state of the phenol compound. Particularly when reaction is conducted by the fixed-bed flow method, there may be provided a screen or the like as needed on the upper portion and/or the lower portion ofthe apparatus so that the gel catalyst beads as the filled catalyst for producing a bisphenol compound do not flow outside the apparatus whereas only reaction solution can circulate. The raw materials may be flown from the upper to the lower portion of the reaction apparatus (down-flow system) or from the lower to the upper portion thereof (up-flow system). Regarding the fixed-bed flow method, there are known problems. For example, the up-flow system easily causes catalyst fluidization and catalyst outflow due to the fluidization, whereas the down-flow system easily causes pressure loss (differential pressure). Therefore, in consideration of those problems, an appropriate method can be selected as occasion demands. The pressure loss can be selected in an appropriate range according to the amount of catalyst, LHSV, capabilities of the apparatus to be used, and the like.

Reaction temperature in the method for producing a bisphenol compound ofthe present invention is usually a temperature at which the reaction solution is not solidified and can be present in a liquid form. For example, when the phenol compound is phenol, the reaction temperature is 40°C or higher, and preferably 50°C or higher. The higher the reaction temperature, the more advantageous it is in terms ofreaction speed. From the viewpoint of the heat resistant temperature of cation exchange resin, reaction is preferably conducted under conditions in which the maximum temperature inside the reactor is 120°C or lower, and particularly 100°C or lower. When the reaction temperature is higher, detachment of strongly acidic group such as sulfonic acid group is partially caused by decomposition even at a temperature lower than the heat-resistant temperature of cation exchange resin. Thus, from such a viewpoint, it is preferable to set the reaction temperature as low as possible, although too low temperature may cause solidification of a produced bisphenol compound.

When the catalyst for producing a bisphenol compound of the present invention is used in the condensation reaction of a phenol compound and a carbonyl compound, water contained in the gel catalyst beads is preferably removed before using the catalyst in the reaction, since the water left inside the gel catalyst beads becomes a reaction inhibitory factor. For example, preferably, the water inside the gel catalyst beads is removed by contacting it with a phenol compound as the raw material. Such a treatment shortens an introduction period when the reaction starts, so that the catalyst can be immediately used in the reaction.

The phenol compound used in the method for producing a bisphenol compound according to the present invention (phenol compound other than the phenol compound recovered and used in the below-described bisphenol production process) may be used as it is if it has high purity. However, the phenol compound is preferably used after purification. The method for purifying the phenol compound is not specifically restricted. For example, there may be mentioned a method in which a phenol compound is brought into contact with an acidic catalyst such as a common sulfonic acid cation exchange resin at 40 to 110°C to convert an impurity contained in the phenol compound to a heavy material, and then distillation is performed to remove the heavy fraction. Usually, the purified phenol compound thus obtained can be fed into the reactor to be used as a reaction raw material.

In addition, as the phenol compound used in the method for producing a bisphenol compound according to the present invention, a phenol compound recovered in the process for producing a bisphenol compound may be recycled to be used. Examples of the phenol compound to be recycled include a phenol solution in which an intended bisphenol compound has been separated from the reaction solution. Specifically, there may be mentioned a phenol solution, which is generally called "mother liquid", obtained when separating a bisphenol compound by a method in which the bisphenol compound is solidified by crystallization or the like to be separated into a solid and a liquid in a solid-liquid separation process, and as another one, a phenol solution obtained from separation by distillation or the like. The phenol compound to be recycled is not restricted to them.

The phenol compound thus purified can also be used by an intended method according to the process, such as using it as a washing solution for crystal obtained in the solid-liquid separation process to be recycled together with the mother liquid into the reactor.

In that case, preferably, after the total amount or a part ofthe phenol compound is separated to be processed with an acid or alkaline catalyst, impurities such as a heavy fraction are removed and a bisphenol compound is recovered, followed by its use as a raw material for condensation reaction. When recycling the phenol compound recovered in the process to use it as the washing solution for crystal obtained in the solid-liquid separation process, it is preferably used alter purification.

On a small scale, such as a laboratory or the like, only a high purity phenol compound purified as a phenol

compound used as a raw material may be used. However, on an industry-level scale, usually, it is economically advantageous to recycle a phenol compound recovered in the process for use.

The reaction solution produced by the above method includes, along with an intended bisphenol compound, too much phenol compound, an unreacted carbonyl compound, an impurity generated in condensation reaction, and the like. Thus, it is necessary to extract the intended bisphenol compound from the solution containing them. The method for separating a bisphenol compound as an intended substance from the reaction solution to purify is not specifically restricted and the separation and purification is conduced based on a known method. A typical example of the separation and purification method will be described below using an example of bisphenol A as an intended substance.

Following the above condensation reaction, in a low boiling point component separation process, the reaction solution obtained by the condensation reaction is separated into a component including bisphenol A and phenol and a low boiling point component including water as a by-product ofthe reaction, an unreacted acetone, and the like. The low boiling point component separation process is preferably conducted by a method of separating the low boiling point component by distillation under reduced pressure, and the low boiling point component may include phenol or the like. Regarding the component including bisphenol A and phenol, the concentration of bisphenol A can be adjusted to a desired concentration, as needed, by the removal of phenol by additionally distillation or the like or by the addition of phenol.

Subsequently, in a crystallization process, there is obtained a slurry containing crystal of an adduct of bisphenol A and phenol. The concentration of the bisphenol A in the component containing the bisphenol A and phenol subjected to the crystallization process is preferably 10 to 30% by weight in terms of easiness of treatment of slurry to be obtained or the like. In addition, examples of the crystallization method include a method of directly cooling the component containing bisphenol A and phenol; a method of mixing another solvent such as water and evaporating the solvent to cool down; a method of additionally removing phenol for concentration, and a method of combining those methods. To obtain an adduct having an intended purity, crystallization may be conducted once or twice or more. The slurry obtained in the crystallization process is solid-liquid separated into crystal of an adduct and a mother liquid by pressure-reduced filtration, pressurized filtration, centrifugal filtration, or the like, whereby the crystal of the adduct ofbisphenol A and phenol is recovered In the above crystallization process, by crystallization, crystal ofbisphenol A may be directly obtained by adjusting the composition and crystallization conditions of the component containing bisphenol A and phenol subjected to crystallization.

In a subsequent dephenolization process, the crystal of the adduct obtained in the solid-liquid separation process is melted, and then, from the melted crystal, phenol is removed by any one method of flush-distillation, thin-film distillation, and steam stripping or by combining the methods thereof, thereby obtaining a molten bisphenol A having high purity. If desired, the removed phenol may be purified to be used for reaction or washing of the crystal of the adduct obtained in the solid-liquid separation process. The obtained molten bisphenol A having high purity is solidified in a granulation process, in which it is easy and preferable to obtain small spherical bisphenol A prills by ejecting the molten bisphenol A from nozzles to contact it with a cooling gas. Alternatively, without the dephenolization process, crystallization can be conducted again to obtain only bisphenol A by crystallization from the crystal of the adduct obtained in the solid-liquid separation process.

Furthermore, to prevent impurity accumulation in the system, at least a part ofthe mother liquid separated in the solid-liquid separation process can be processed in an impurity processing process. For example, as an economically preferable method, alkali or acid is mixed with the mother liquid to be heated; the mixture product is subjected to distillation to separate into a light fraction and a heavy fraction; and the light fraction is subjected to rebonding reaction using an acidic catalyst or the like to use it in reaction Thus, by purging the heavy fraction outside the system, impurity accumulation can be prevented, so that product purity can be improved. In addition, by conducting crystallization after isomerization of at least a part ofthe mother liquid with an acid catalyst, the recovery rate ofbisphenol A can be improved.
Regarding the low boiling point component obtained in the low boiling point component separation process, the unreacted acetone can be separated and recovered in an acetone circulation process to circulate the recovered acetone into the reaction process.

## EXAMPLES

Hereinafter, the present invention will be described in more detail by way of Examples. However, the present invention is not limited to the Examples below without departing from the gist of the present invention.
In the description below, the term "parts" means "parts by weight".

### Example 1

#### (1) Production of Copolymer (Gel Beads)

As shown in Fig. 1, a liquid droplet production apparatus equipped with an underwater speaker as a vibrator

and a polymerization reaction apparatus were used to produce spherical gel beads (hereinafter may be referred to as "copolymer") having a uniform particle diameter.

The liquid droplet production apparatus 1 is provided with a liquid droplet production tank 3 holding an aqueous medium 2 forming a continuous phase, a hydrophobic liquid storage tank 5 holding a hydrophobic liquid 4 that does not mix with the aqueous medium 2, and a hydrophobic liquid supply pipe 6 for supplying the hydrophobic liquid 4 stored in the hydrophobic liquid storage tank 5 to the liquid droplet production tank 3. In addition, the liquid droplet production apparatus 1 is also provided with a nozzle member 7 that is in contact with the aqueous medium 2 and has ejection holes 11 for ejecting the hydrophobic liquid 4 supplied from the hydrophobic liquid supply pipe 6, an underwater speaker (underwater acoustic apparatus) 8 as a vibrating means for mechanically applying vibration to the aqueous medium 2 in the liquid droplet production tank 3, an aqueous medium storage tank 9 for storing the aqueous medium 2, and an aqueous medium supply pipe 10 for supplying the aqueous medium 2 stored in the aqueous medium storage tank 9 to the liquid droplet production tank 3. As used herein, reference numeral 12 denotes a hydrophobic liquid ejection/storage tank, reference numerals 13 and 14, respectively, denote a hydrophobic liquid supply pump and an aqueous medium supply pump, respectively.

The nozzle member 7 used was, as shown in Fig. 2, a nozzle member with 490 ejection holes 11 having a diameter of 0.1 mm arranged in a ring shape on a round plate with an outer diameter of 100 mm.

Furthermore, the polymerization reaction apparatus 16 shown in Fig. 1 has a polymerization reaction tank 17 to which liquid droplets 15 in the liquid droplet production tank 3 ofthe liquid droplet production apparatus 1 are transferred together with the aqueous medium 2 to conduct polymerization reaction without causing cohesion and crushing of the liquid droplets 15, and a hydrophobic liquid droplet transfer pipe 18 for transferring the liquid droplets 15 and the aqueous medium 2 together from the liquid droplet production tank 3 to the polymerization reaction tank 17 without causing the cohesion and crushing ofthe liquid droplets 15.

In the liquid droplet production apparatus 1, into the aqueous medium 2 hold in the liquid droplet production apparatus 3 to form a continuous phase, the hydrophobic liquid 4 transferred from the hydrophobic liquid storage tank 5 via the hydrophobic liquid supply pipe 6 by the supply pump 13 is ejected from the ejection holes 11 provided on the nozzle member 7, thereby allowing the formation of an ejection flow of the hydrophobic liquid 4. In this case, for example, by vibrating the aqueous medium 2 using the underwater speaker 8, the ejection flow can be crushed to form hydrophobic liquid droplets 15 having a uniform particle diameter. In addition, by supplying the aqueous medium 2 stored in the aqueous medium storage tank 9 into the liquid droplet production tank 3 by the supply pump 14, a flow of the aqueous medium 2 can be formed in the liquid droplet production tank 3, so that the flow can move hydrophobic liquid droplets 15 produced.

In other words, at an inside lower portion of the liquid droplet production tank 3, the hydrophobic liquid ejection/ storage tank 12 is located, and on a top portion thereof is attached the nozzle member 7 having the ejection holes 11 open toward into the aqueous medium 2 to eject the hydrophobic liquid 4. Thus, the hydrophobic liquid 4 supplied by the supply pump 13 via the hydrophobic liquid supply pipe 6 from the hydrophobic liquid storage tank 5 is stored in the liquid ejection/storage tank 12 to be ejected upward upright from the ejection holes 11 provided on the nozzle member 7. As shown in Fig. 2, on the nozzle member 7, the plurality of ejection holes 11 for ejecting the hydrophobic liquid 4 are arranged at a predetermined interval. The diameter of the ejection holes 11 is determined according to an intended liquid droplet size.

The liquid droplet production tank 3 is connected to the polymerization reaction tank 17 by the liquid droplet transfer pipe 18. Thus, by the flow of the aqueous medium 2 in the liquid droplet production tank 3 formed by supplying the aqueous medium 2 into the liquid droplet production tank 3 from the aqueous medium storage tank 9, the hydrophobic liquid droplets 15 produced in the liquid droplet production tank 3 are continuously transferred together with the aqueous medium 2 into the polymerization reaction tank 17 to be subjected to polymerization reaction.

In the present Example, first, as the aqueous medium 2, an aqueous solution containing 0.05% by weight of polyvinyl alcohol was filled into the liquid droplet production tank 3 and the polymerization reaction tank 17 from the aqueous medium storage tank 9. The polyvinyl alcohol solution was heated to 40°C and maintained until the start of polymerization reaction. Meanwhile, a polymerizable monomer mixture solution containing 96 parts of styrene containing benzoyl peroxide as a polymerization initiator and 4 parts of divinyl benzene was ejected as a hydrophobic liquid into the liquid droplet production tank 3 through the ejection holes 11 of the nozzle member 7 from the hydrophobic liquid storage tank 5 at a flow rate of 1.19 mL/min/hole. In this case, to crush the ejection flow of the polymerizable monomer mixture solution into the liquid droplets 15 having a uniform particle diameter, a vibration of 2100 Hz was applied to the ejection flow by the underwater speaker 8. The liquid droplets 15 of the polymerizable monomer mixture solution thus obtained had a mean particle diameter of 0.26 mm and a uniformity coefficient of 1.02. The mean particle diameter and the uniformity coefficient ofthe liquid droplets 15 were calculated by taking enlarged photos ofthe droplets and obtaining a particle diameter distribution using an image analysis method.

The produced liquid droplets 15 were transferred to the polymerization reaction tank 17, along with a sending flow ofthe aqueous medium 2. Next, the liquid droplets 15 were stirred in the polymerization reaction tank 17 at a rotation rate that

did not cause the cohesion or crushing ofthe liquid droplets 15 and simultaneously heated at 75°C for 8 hours to conduct polymerization, whereby a copolymer (crosslinking degree: 4%) was obtained.
A slurry ofthe obtained copolymer was separated into a solid fraction and a liquid fraction by a centrifugal separator to recover a copolymer in a state containing no polyvinyl alcohol solution. The copolymer was obtained as spherical particles with a mean particle diameter of 0.25 mm and a uniformity coefficient of 1.02.

The mean particle diameter and the uniformity coefficient of the copolymer were calculated from a particle diameter distribution measured by a sieving method described in "Manual 1 of DIAION: Ion Exchange Resin · Synthetic Absorbent" (published by Mitsubishi Chemical Corp., 4th revised edition, issued on Oct. 31, 2007, pp. 140-141) using the following formulas:

$$\text{Mean particle diameter} = \text{diameter equivalent to 50\% of cumulative volume of resin.}$$

$$\text{Uniformity coefficient} = \text{diameter in which cumulative volume of larger particles is}$$

$$\text{equivalent to 40\% / diameter in which cumulative volume of larger particles is equivalent to 90\%.}$$

(2) Production of Gel Catalyst Beads

180 g ofthe obtained copolymer was placed in a 1L four-necked flask, and then, 198 g of nitrobenzene was added thereto. The mixture was heated and stirred at 70°C for 1.5 hours to cause the copolymer to swell. After cooling it down, 324 g of nitrobenzene, 360 g of 98% by weight of sulfuric acid, and 189 g of turning sulfuric acid were added to increase the temperature of the mixture up to 70°C. The mixture was heated for 4 hours to increase the temperature up to 105°C, and then it was maintained for 3 hours.
After reaction, a large amount of water was added to dilute and remove the sulfuric acid in the flask and then, desalted water was added. The reaction product was heated and stirred, followed by evaporation of nitrobenzene. The obtained resin was washed with desalted water to obtain gel catalyst beads (hereinafter may be referred to as "strongly acidic cation exchange resin").
There were calculated an exchange capacity, a pressure loss, a mean particle diameter, a uniformity coefficient, and a content of catalyst beads with particle diameters of 30 to 650 μm of the obtained strongly acidic cation exchange resin. The results were shown in Table 1A.
The content of catalyst beads with particle diameters of 30 to 650 μm was calculated from a particle diameter distribution obtained by the same sieving method as in the above-described copolymer. Furthermore, the exchange capacity and the pressure loss were obtained as follows.

<Measurement of Exchange Capacity>

In a washed flask are placed 0.50 g of the wet (hereinafter may be referred to as "g-wet") strongly acidic cation exchange resin, 0.25 g of sodium chloride, and 20 mL of desalted water. After stirring the mixture at room temperature for approximately 30 minutes, a commercially available methyl red-methylene blue mixed indicator solution was titrated with 0.1N sodium hydroxide solution (force value: fNaOH) to measure a dropped amount (AmL), whereby the exchange capacity was calculated by the following formula:

$$\text{Exchange capacity (meq/g-wet)} = A \times 0.1 \times \text{fNaOH}/0.50$$

<Measurement of Pressure Loss>

(Pretreatment)

Between glass filters 21 and 22 of a glass column 19 having an inner diameter of 1 cm and an entire length of 50 cm as shown in Fig. 3 was filled 30. 0 mL of the wet strongly acidic cation exchange resin in a water slurry state. After removing water in the column 19 through a column lower valve 25 and a column lower line 26, a water/phenol

mixed solution (weight ratio: 10/90) was fed at a flow rate of 5 mL/min at room temperature for approximately 1 hour in an upward flow from the valve 25 and the line 26. After removing the water/phenol mixed solution in the column through the valve 25 and the line 26, phenol of 60°C was fed at the flow rate of 5 mL/min for approximately 2 hours through the valve 25 and the line 26 in the upward flow.

(Measurement of Pressure Loss)

Through a column upper valve 24 and a column upper line 23 in a downward flow, phenol of60°C was fed at a flow rate of 85 mL/min to measure a pressure loss (kPa/m) per 1 m of the filled layer in the strongly acidic cation exchange resin filled layer 20. The pressure loss in this case was carried out by the measurements of a liquid pressure at an inlet portion of the column 19 and a liquid pressure at an outlet portion thereof, using a digital pressure sensor AP-V80 manufactured by Keyence Corporation.

(3) Modification of Gel Catalyst Beads

In a 200 mL four-necked flask with a nitrogen gas-introducing pipe were placed 20.0 g-wet of the strongly acidic cation exchange resin in the wet state produced in the above process and approximately 40 mL of desalted water of 60°C to wash the strongly acidic cation exchange resin. The washing solution was discarded by decantation, and again, approximately 40 mL of desalted water of 60°C was introduced to repeat the washing operation three times. Next, after discarding the washing solution, approximately 40 mL of desalted water was added and substitution with nitrogen in the flask was conducted. To the obtained product was added 0.74 g (5.32 millimole) of 4-(2-mercaptoethyl)pyridine as a modifying agent (promoter) all at once under stirring. Then, the mixture product was further stirred at room temperature for 2 hours to conduct modification processing. After completing the processing, the obtained modified cation exchange resin was washed with desalted water to obtain a 4-(2-mercaptoethyl)pyridine-modified strongly acidic cation exchange resin catalyst.

The modification rate was calculated according to the following formula, based on the amount of the strongly acidic cation exchange resin used for modification, the amount ofthe added modifying agent (4-(2-mercaptoethyl)pyridine), and the amount of sulfonic acid group in the strongly acidic cation exchanger resin obtained by titration. The result was shown in Table 1A. The amount of sulfonic acid group in the strongly acidic cation exchanger resin corresponds to the above exchange capacity.

$$\text{Modification rate (\%)} = [(\text{amount by mole (millimole) of added modifying agent}) / \{(\text{amount of sulfonic acid group in the strongly acidic cation exchange resin (meq/g-wet)}) \times \text{weight of the strongly acidic cation exchange resin (g- wet) used for modification})\}] \times 100.$$

(4) Production of Bisphenol A

In a 200 mL glass flask equipped with a nitrogen-introducing pipe, a motor stirrer, and a Dimroth condenser was placed 3.0 g-wet of a modified strongly acidic cation exchange resin catalyst (modification rate: 15.3%) obtained in the above (3). The catalyst was washed with approximately 100 mL of phenol of 70°C until the water content of the washing solution became 0.1 % by weight or less. Next, in the flask was placed 90.0 g of 70°C phenol, and nitrogen was introduced. Under stirring, 4.27 g of acetone was added to start reaction. After starting the reaction, the reaction solution was collected chronologically to be analyzed by gas chromatography under such conditions as follows, whereby a 4,4'-bisphenol A yield and a 4,4'-isomer/2,4'-isomer ratio were calculated from analysis values by the following formulas. Table 1A showed a 4,4-bisphenol A yield obtained at a lapse of 60 minutes after the start ofreaction and a 4,4'-isomer/2,4'-isomer ratio obtained at a point in time when the 4,4-bisphenol A yield had reached 50%.
The terms "4,4'-bisphenol A" and "4,4'-isomer" refer to 2,2-bis(4-hydroxyphenyl)propane, and the term "2,4'-isomer" refers to 2-(2-hydroxyphenyl)-2-(4-hydroxyphenyl)propane.

<Gas Chromatography Analysis>

Gas chromatography: "GC-2014" manufactured by Shimadzu Corporation
Column: "Ultra Performance Capillary Column Ultra 2 (Cross-linked 5% phenylmethyl silicone) 25 m × 0.32 mm × 0.52 ×m" manufactured by Agilent Technologies Inc.

Detector: FID
Carrier gas: He
4,4'-bisphenol A yield (%) = [(amount by mole of produced 4,4'-bisphenol A) /(amount by mole of acetone used in reaction)] × 100
4,4'-isomer/2,4'-isomer ratio = [(amount by mole of produced 4,4'-bisphenol A)/(amount by mole of produced 2,4'-bisphenol A)]

Example 2

(1) Production of Gel Beads

Liquid droplets and a copolymer were produced in the same manner as in Example 1, except that, by using a nozzle member with 345 ejection holes 11 having a diameter of 0.125 mm arranged on a round plate having an outer diameter of 100 mm as a nozzle member, a polymerizable monomer mixed liquid was ejected from the ejection holes of the nozzle member at a flow rate of 1.54 mL/min/hole to apply a vibration of 1400 Hz by the underwater speaker. The liquid droplets obtained at that time had a mean particle diameter of 0.32 mm and a uniformity coefficient of 1.01. In addition, the copolymer obtained was spherical particles having a mean particle diameter of 0.29 mm and a uniformity coefficient of 1.02.

(2) Production of Gel Catalyst Beads

Using the above copolymer, a strongly acidic cation exchange resin was produced in the same method as in Example 1, and similarly, there were obtained an exchange capacity, a pressure loss, a mean particle diameter, a uniformity coefficient, and a content of catalyst beads having particle diameters of 30 to 650 μm in the obtained strongly acidic cation exchange resin. The results were shown in Table 1A.

(3) Modification of Gel Catalyst Beads

The strongly acidic cation exchange resin catalyst was modified in the same method as in Example 1 and modification rate was calculated. The result was shown in Table 1A.

(4) Production of Bisphenol A

Using the modified strongly acidic cation exchange resin, bisphenol A was produced in the same method as in Example 1, and the results of gas chromatography analysis were shown in Table 1A.

Example 3

Liquid droplets and a copolymer were produced in the same manner as in Example 1, except that, by using the nozzle member where 345 ejection holes 11 having a diameter of 0.125 mm were arranged on the round plate with an outer diameter of 100 mm as the nozzle member, a polymerizable monomer mixed liquid was ejected from the ejection holes of the nozzle member at the flow rate of 1.54 mL/min/hole to apply a vibration of 900 Hz by the underwater speaker. The liquid droplets obtained at that time had a mean particle diameter of 0.37 mm and a uniformity coefficient of 1.02. In addition, the copolymer obtained was spherical particles having a mean particle diameter of 0.35 mm and a uniformity coefficient of 1.02.
Using the above copolymer, a strongly acidic cation exchange resin was produced in the same method as in Example 1, and similarly, there were obtained exchange capacity, pressure loss, mean particle diameter, uniformity coefficient, and the content of catalyst beads having a particle diameter of 30 to 650 μm in the obtained strongly acidic cation exchange resin. The results were shown in Table 1A.
The strongly acidic cation exchange resin was modified in the same method as in Example 1 and modification rate was calculated. The result was shown in Table 1A. In addition, using the modified strongly acidic cation exchange resin, bisphenol A was produced in the same method as in Example 1, and results of gas chromatography analysis were shown in Table 1A.

Example 4

In a 3 L four-necked flask equipped with a nitrogen-introducing pipe and a cooling pipe were placed 2316 mL of desalted water and 27.5 mL of 6% by weight of polyvinyl alcohol aqueous solution. Then, nitrogen was introduced to

remove dissolved oxygen.
Meanwhile, a polymerizable monomer mixed liquid was prepared by adding 306.8 g of styrene, 23.2 g of divinylbenzene having a purity of 57% by weight containing 43% by weight of ethylvinylbenzene, and 0.43 g of benzoyl peroxide having a water content of 25% by weight. The polymerizable monomer mixed liquid was placed in the above flask and stirred at 140 rpm to obtain a suspension, which was then stirred for 30 minutes at room temperature. After stirring, the temperature of the liquid was increased up to 75 °C, and the liquid was maintained at 75 °C for 8 hours to conduct polymerization reaction. After polymerization, the polyvinyl alcohol aqueous solution was removed to obtain a copolymer. The copolymer was subjected to elutriation to remove small particles.
Using the above copolymer, a strongly acidic cation exchange resin was produced in the same method as in Example 1, and similarly, there were obtained exchange capacity, pressure loss, mean particle diameter, uniformity coefficient, and a content of catalyst beads having particle diameters of 30 to 650 μm in the obtained strongly acidic cation exchange resin. The results were shown in Table 1A.
The strongly acidic cation exchange resin catalyst was modified in the same method as in Example 1 and modification rate was calculated. The result was shown in Table 1A. Additionally, using the modified strongly acidic cation exchange resin, bisphenol A was produced in the same method as in Example 1, and results of gas chromatography analysis were shown in Table 1A.

Example 5

A polyvinyl alcohol aqueous solution and a polymerizable monomer mixed liquid were prepared in the same method as in Example 4 and stirred at 130 rpm to obtain a suspension. The suspension was maintained at 75 °C for 8 hours to conduct copolymerization reaction.
After polymerization, the polyvinyl alcohol aqueous solution was removed to obtain a copolymer. The copolymer was subjected to elutriation to remove small particles.
Using the above copolymer, a strongly acidic cation exchange resin was produced in the same method as in Example 1, and similarly, there were obtained exchange capacity, pressure loss, mean particle diameter, uniformity coefficient, and a content of catalyst beads having particle diameters of 30 to 650 μm in the obtained strongly acidic cation exchange resin. The results were shown in Table 1A.
The strongly acidic cation exchange resin catalyst was modified in the same method as in Example 1 and modification rate was calculated. The result was shown in Table 1A. Furthermore, using the modified strongly acidic cation exchange resin, bisphenol A was produced in the same method as in Example 1, and results of gas chromatography analysis were shown in Table 1A.

Example 6

A polyvinyl alcohol aqueous solution and a polymerizable monomer mixed liquid were prepared in the same method as in Example 4 and stirred at 120 rpm to obtain a suspension. The suspension was maintained at 75 °C for 8 hours to conduct copolymerization reaction.
After polymerization, the polyvinyl alcohol aqueous solution was removed to obtain a copolymer. The copolymer was subjected to elutriation to remove small particles.
Using the above copolymer, a strongly acidic cation exchange resin was produced in the same method as in Example 1, and similarly, there were obtained exchange capacity, pressure loss, mean particle diameter, uniformity coefficient, and a content of catalyst beads having particle diameters of 30 to 650 μm in the obtained strongly acidic cation exchange resin. The results were shown in Table 1A.
The strongly acidic cation exchange resin catalyst was modified in the same method as in Example 1 and modification rate was calculated. The result was shown in Table 1A. Additionally, using the modified strongly acidic cation exchange resin, bisphenol A was produced in the same method as in Example 1, and results of gas chromatography analysis were shown in Table 1A.

Comparative Example 1

As a strongly acidic cation exchange resin, there was prepared a gel-type strongly acidic cation exchange resin having a crosslinking degree of 4% (product name: DIAION (registered trademark) SK 104) manufactured by Mitsubishi Chemical Co., Ltd. Table 1A shows the exchange capacity, mean particle diameter, uniformity coefficient, the content of catalyst beads having particles diameters of 30 to 650 μm in the strongly acidic cation exchange resin. In addition, pressure loss measured in the same manner as in Example 1 is as shown in Table 1A.
The strongly acidic cation exchange resin catalyst was modified in the same method as in Example 1 and modification rate was calculated. Table 1A shows the result. Additionally, using the modified strongly acidic cation exchange resin,

bisphenol A was produced in the same method as in Example 1 and an analysis result of gas chromatography was shown in Table 1A.

Example 7

In the same manner as in Example 1 except that as a modifier (promoter), instead of 4-(2-mercaptoethyl) pyridine, a solution of 0.21 g (2.72 millimole) of2-methylcaptoethyl amine hydrochloride/10 mL of desalted water was dropped in 20 minutes at room temperature under stirring, 10.0 g-wet of a wet strongly acidic cation exchange resin produced in Example 1 was used to prepare a 2-mercaptoethylamine-modified strongly acidic cation exchange resin catalyst, and modification rate was calculated. Table 1B shows the result.
Using the 2-mercaptoethylamine-modified strongly acidic cation exchange resin catalyst, bisphenol A was produced in the same method as in Example 1, and the analysis result of gas chromatography was shown in Table 1B.

Comparative Example 2

A 2-mercaptoethylamine-modified strongly acidic cation exchange resin catalyst was prepared in the same manner as in Example 7 except for the use ofthe gel-type strongly acidic cation exchange resin having a crosslinking degree of 4% (product name: DIAION (registered trademark) SK 104) manufactured by Mitsubishi Chemical Co., Ltd, as a strongly acidic cation exchange resin, and modification rate was calculated and the result was shown in Table 1B.
Using the 2-mercaptoehtylamine-modified strongly acidic cation exchange resin catalyst, bisphenol A was produced in the same method as in Example 1 to analyze by gas chromatography, and the analysis results were shown in Table 1B.

Example 8

A 2-(2-mercaptoethyl)pyridine-modified strongly acidic cation exchange resin catalyst was prepared in the same manner as in Example 1 except for the use of 2-(2-mercaptoethyl)pyridine instead of 4-(2-mercaptoethyl)pyridine, as a modifier (promoter). Modification rate was calculated and the result was shown in Table 1C.
Bisphenol A was produced in the same manner as in Example 1 except for the use of the 2-(2-mercaptoethyl)pyridine-modified strongly acidic cation exchange resin catalyst. Results of gas chromatography analysis were shown in Table 1C.

Comparative Example 3

A 2-(2-mercaptoethyl)pyridine-modified strongly acidic cation exchange resin catalyst was prepared in the same manner as in Example 8 except for the use of the gel-type strongly acidic cation exchange resin having a crosslinking degree of 4% (product name: DIAION (registered trademark) SK 104) manufactured by Mitsubishi Chemical Co., Ltd, as a strongly acidic cation exchange resin. Modification rate was calculated and the result was shown in Table 1C.
Bisphenol A was produced in the same manner as in Example 1 except for the use of the 2-(2-mercaptoethyl)pyridine-modified strongly acidic cation exchange resin catalyst and results of gas chromatography analysis were shown in Table 1C.

[Table 1A]

| | Ex.1 | Ex.2 | Ex.3 | Ex.4 | Ex.5 | Ex.6 | Comp. Ex.1 |
|---|---|---|---|---|---|---|---|
| Mean particle diameter (μm) | 420 | 521 | 601 | 439 | 511 | 592 | 730 |
| Uniformity coefficient | 1.03 | 1.01 | 1.02 | 1.30 | 1.35 | 1.27 | 1.48 |
| Degree of crosslinking (%) | 4 | 4 | 4 | 4 | 4 | 4 | 4 |
| Exchange capacity (meq/g-wet) | 1.75 | 1.72 | 1.76 | 1.75 | 1.72 | 1.72 | 1.67 |
| Modification rate (%) | 15.3 | 15.3 | 15.2 | 15.3 | 15.2 | 15.5 | 15.9 |
| Pressure loss (kPa/m) | 425 | 272 | 200 | 605 | 432 | 251 | 107 |
| Content of gel catalyst beads having particle diameters of 30 to 650 μm (% by volume) | 100 | 99 | 99 | 100 | 97 | 77 | 40 |
| 4,4'-bisphenol A yield at 60 min after start of reaction (%) | 60.4 | 56.9 | 51.8 | 60.2 | 59.4 | 50.3 | 44.5 |

(continued)

| | Ex.1 | Ex.2 | Ex.3 | Ex.4 | Ex.5 | Ex.6 | Comp. Ex.1 |
|---|---|---|---|---|---|---|---|
| 4,4'-isomer/2,4'-isomer ratio at 50% yield of 4,4'-bisphenol A | 57 | 55 | 55 | 57 | 56 | 54 | 51 |

[Table 1B]

| | Ex. 7 | Comp. Ex. 3 |
|---|---|---|
| Mean particle diameter (μm) | 420 | 730 |
| Uniformity coefficient | 1.03 | 1.48 |
| Degree of crosslinking (%) | 4 | 4 |
| Exchange capacity (meq/g-wet) | 1.75 | 1.67 |
| Modification rate (%) | 15.8 | 15.0 |
| Content of gel catalyst beads having particle diameters of 30 to 650 μm (% by volume) | 100 | 40 |
| 4,4'-bisphenol A yield at 60 min after start of reaction (%) | 42.0 | 35.4 |
| 4,4'-isomer/2,4'-isomer ratio at 50% yield of 4,4'-bisphenol A | 38 | 36 |

[Table 1C]

| Mean particle diameter (μm) | Ex. 8 | Comp. Ex. 3 |
|---|---|---|
| Uniformity coefficient | 420 | 730 |
| Degree of crosslinking (%) | 1.03 | 1.48 |
| Exchange capacity (meq/g-wet) | 4 | 4 |
| Modification rate (%) | 1.75 | 1.67 |
| Mean particle diameter (μm) | 16.3 | 16.1 |
| Content of gel catalyst beads having particle diameters of 30 to 650 μm (% by volume) | 100 | 40 |
| 4,4'-bisphenol A yield at 60 min after start ofreaction (%) | 56.2 | 43.5 |
| 4,4'-isomer/2,4'-isomer ratio at 50% yield of 4,4'-bisphenol A | 51 | 45 |

The above results showed that, in the catalyst for producing a bisphenol compound according to the present invention, the gel catalyst beads having particle diameters 30 to 650 μm accounted for 50% or more of total beads, and as the content ofthe above beads increased, the 4,4'-bisphenol A yield and the ratio 4,4'-isomer/2,4'-isomer ratio were improved, thereby allowing an intended 4,4'-bisphenol A to be efficiently produced. It was also shown that, when using, as a promoter, 4-(2-mercaptoethyl)pyridine and 2-(2-mercaptoethyl)pyridine, there was obtained a more significant effect on the improvement in the 4,4'-bisphenol A yield and the 4,4'-isomer/2,4'-isomer ratio, thereby allowing 4,4'-bisphenol A to be more efficiently produced.

Example 9

(1) Production of Copolymer

In a 3 L four-necked flask equipped with a nitrogen-introducing pipe and a cooling pipe were placed 878 mL of desalted water, 675 mL of 6% by weight of polyvinyl alcohol aqueous solution, and 23 mL of 0.1% sodium nitrite solution, and nitrogen was introduced to remove dissolved oxygen.
Meanwhile, a polymerizable monomer mixed liquid was prepared by adding 362.9 g of styrene, 11.92 g of divinylbenzene

having a purity of 63% by weight containing 37% by weight of ethylvinylbenzene, and 2.5 g of benzoyl peroxide having a water content of 25% by weight. The polymerizable monomer mixed liquid was placed in the above flask and stirred at 250 rpm to obtain a suspension, which was stirred at 30°C for 30 minutes. After stirring, the temperature of the suspension was increased up to 80°C, and it was maintained at 80°C for 8 hours to conduct copolymerization reaction. After polymerization, the polyvinyl alcohol aqueous solution was removed and the copolymer was washed with 2 L of desalted water. After washing, again in the flask were added the copolymer and 1.5 L of desalted water, and the mixture was stirred at 150 to 200 rpm and at the same time, heated at 100°C for 2 hours. The resultant copolymer was subjected to elutriation to remove particles having diameters of 10 μm or less and particles having diameters of 200 μm or more.

(2) Production of Gel Catalyst Beads

A strongly acidic cation exchange resin was produced in the same manner as in Example 1 except that, in a 3 L four-necked flask was placed 179 g of the obtained copolymer, and 540 mL of nitrobenzene was added; the mixture was heated and stirred at 70°C for 1 hour to cause the copolymer to swell; after cooling it down, 359 g of 98% by weight of sulfuric acid and 189 g of fuming sulfuric acid were added and the temperature of the mixture was increased up to 60°C to heat it for 2 hours; and the temperature thereof was increased up to 90°C to maintain the reaction mixture for 5 hours. The obtained strongly acidic cation exchange resin was subjected to elutriation in a range of 150 to 210 μm. Then, in the same manner as in Example 1, there were obtained exchange capacity, mean particle diameter, uniformity coefficient, and a content of gel catalyst beads having particle diameters of 650 μm or less in the strongly acidic cation exchange resin. The results were shown in Table 2.

(3) Modification of Gel Catalyst Beads

Modification of the strongly acidic cation exchange resin catalyst was conducted in the same method as in Example 1 and modification rate was calculated. The result was shown in Table 2.

(4) Production of Bisphenol A

Using the modified strongly acidic cation exchange resin, bisphenol A was produced and analyzed by gas chromatography in the same method as in Example 1. From the analysis values, there were calculated the 4,4'-bisphenol A yield and the 4,4'-isomer/2,4'-isomer ratio in the same manner as in Example 1. Additionally, TOF ($hr^{-1}$) was calculated by the following formula and the result was shown in Table 2.

$$\text{TOF (hr}^{-1}\text{)} = [\text{amount by mole of 4,4'-bisphenol A produced at 60 minutes after starting of reaction}]/[(\text{amount by mole of all sulfuric acid groups of the strongly acidic cation exchange resin catalyst}) \times \text{reaction time}]$$

Example 10

The strongly acidic cation exchange resin obtained in Example 9 was subjected to elutriation in a range of 100 to 170 μm, and as in Example 1, there were obtained exchange capacity, mean particle diameter, uniformity coefficient, and a content of catalyst beads having particle diameters of 650 μm or less in the obtained strongly acidic cation exchange resin. The results were shown in Table 2.
Modification ofthe strongly acidic cation exchange resin catalyst was conducted in the same method as in Example 1. Modification rate was calculated and the result was shown in Table 2.
Using the modified strongly acidic cation exchange resin, bisphenol A was produced in the same method as in Example 1. Results of gas chromatography analysis were shown in Table 2.

Example 11

The strongly acidic cation exchange resin obtained in Example 9 was subjected to elutriation in a range of 25 to 120 μm As in Example 1, there were obtained exchange capacity, mean particle diameter, uniformity coefficient, and a content of catalyst beads having particle diameters of 650 μm or less in the obtained strongly acidic cation exchange resin, and the results were shown in Table 2.
Modification ofthe strongly acidic cation exchange resin catalyst was conducted in the same method as in Example 1

and modification rate was calculated. The result was shown in Table 2.
Using the modified strongly acidic cation exchange resin, bisphenol A was produced in the same method as in Example 1. Results of gas chromatography analysis were shown in Table 2.

Example 12

In a 3 L four-necked flask equipped with a nitrogen-introducing pipe and a cooling pipe were placed 878 mL of desalted water, 675 mL of 5% by weight of polyvinyl alcohol aqueous solution, and 23 mL of 0.1 % sodium nitrite aqueous solution. Nitrogen was introduced to remove dissolved oxygen.
Meanwhile, a polymerizable monomer mixed liquid was prepared by adding 351.2 g of styrene, 23.9 g of divinylbenzene having a purity of 63% by weight containing 37% by weight of ethylvinylbenzene, and 2.5 g of benzoyl peroxide having the water content of 25% by weight. The polymerizable monomer mixed liquid was placed in the flask and stirred at 250 rpm to obtain a suspension. The suspension was stirred at 30°C for 30 minutes and then, heated up to 80°C. The reaction solution was maintained at 80°C for 8 hours to conduct copolymerization reaction.
After polymerization, the polyvinyl alcohol aqueous solution was removed and the copolymer was washed with 2 L of desalted water. After washing, again in the flask were added the copolymer and 1.5 L of desalted water, and the mixture was stirred at 150 to 200 rpm and at the same time heated at 100°C for 2 hours. The copolymer thus obtained was subjected to elutriation to remove particles with diameters of 30 μm or less and particles with diameters of 250 μm or more.
A strongly acidic cation exchange resin was produced in the same manner as in Example 1 except that, in a 3 L four-necked flask was placed 179 g of the obtained copolymer and then, 540 mL of nitrobenzene was added; the mixture was heated and stirred at 70°C for 1 hour to cause the copolymer to swell; after cooling it down, 359 g of 98% by weight of sulfuric acid and 189 g of fuming sulfuric acid were added and heated up to 60°C; and the reaction mixture was heated for 2 hours, then heated up to 90°C to be maintained for 5 hours. The obtained strongly acidic cation exchange resin was subjected to elutriation in a range of 180 to 240 μm Then, as in the same manner as in Example 1, there were obtained exchange capacity, mean particle diameter, uniformity coefficient, and a content of catalyst beads having particle diameters of 650 μm or less in the strongly acidic cation exchange resin, and the results were shown in Table 2.
Modification of the strongly acidic cation exchange resin catalyst was conducted in the same method as in Example 1 and modification rate was calculated. The result was shown in Table 2.
Using the modified strongly acidic cation exchange resin, bisphenol A was produced in the same method as in Example 1, and the results of gas chromatography analysis were shown in Table 2.

Example 13

The strongly acidic cation exchange resin obtained in Example 12 was subjected to elutriation in a range of 110 to 170 μm, and as in Example 1, there were obtained exchange capacity, mean particle diameter, uniformity coefficient, and a content of catalyst beads having particle diameters of 650 μm or less, and the results were shown in Table 2.
Modification of the strongly acidic cation exchange resin catalyst was conducted in the same method as in Example 1 and modification rate was calculated. The result was shown in Table 2.
Using the modified strongly acidic exchange resin, bisphenol A was produced in the same method as in Example 1 and results of gas chromatography analysis were shown in Table 2.

Example 14

The strongly acidic cation exchange resin obtained in Example 12 was subjected to elutriation in a range of 60 to 100 μm Then, as in Example 1, there were obtained exchange capacity, mean particle diameter, uniformity coefficient, and a content of catalyst beads having particle diameters of 650 μm or less in the obtained strongly acidic cation exchange resin, and the results were shown in Table 2.
Modification of the strongly acidic cation exchange resin catalyst was conducted in the same method as in Example 1 and modification rate was calculated. Then, the result was shown in Table 2.
Using the modified strongly acidic cation exchange resin, bisphenol A was produced in the same method as in Example 1, and results of gas chromatography analysis were shown in Table 2.

Example 15

(1) Production of Gel beads

In a 3 L four-necked flask equipped with a nitrogen-introducing pipe and a cooling pipe were placed 878 mL

of desalted water, 675 mL of 4% by weight of polyvinyl alcohol aqueous solution, and 23 mL of 0.1% sodium nitrite solution, and nitrogen was introduced to remove dissolved oxygen.
Meanwhile, there was prepared a polymerizable monomer mixed liquid by adding 339.5 g of styrene, 35.8 g of divinybenzene having the purity of 63% by weight containing 37% by weight of ethylvinylbenzene, and 2.5 g of benzoyl peroxide having the water content of 25% by weight. The polymerizable monomer mixed liquid was placed in the above flask and stirred at 250 rpm to obtain a suspension, which was stirred at 30°C for 30 minutes. After stirring, the temperature ofthe suspension was increased up to 80°C, and it was maintained at 80°C for 8 hours to conduct copolymerization reaction. After polymerization, the polyvinyl alcohol aqueous solution was removed and the copolymer was washed with 2 L of desalted water. After washing, again in the flask were added the copolymer and 1.5 L of desalted water, and the mixture was stirred at 150 to 200 rpm and at the same time, heated at 100°C for 2 hours. The resultant copolymer was subjected to elutriation to remove particles with diameters of 20 μm or less and particles with diameters of 250 μm or more.

(2) Production of Gel Catalyst Beads

A strongly acidic cation exchange resin was produced in the same manner as in Example 1 except that, in a 3 L four-necked flask was placed 179 g of the obtained copolymer, and 540 mL of nitrobenzene was added; the mixture was heated and stirred at 70°C for 1 hour to cause the copolymer to swell; after cooling it down, 359 g of 98% by weight of sulfuric acid and 189 g of fuming sulfuric acid were added and the temperature of the mixture was increased up to 60°C to heat the mixture for 2 hours; and the temperature thereofwas increased up to 90°C to maintain it for 5 hours. The obtained strongly acidic cation exchange resin was subjected to elutriation in a range of 190 to 230 μm, and as in Example 1, there were obtained exchange capacity, mean particle diameter, uniformity coefficient, and a content of catalyst beads having particle diameters of 650 μm or less in the obtained strongly acidic cation exchange resin. The results were shown in Table 2.

(3) Modification of Gel Catalyst Beads

Modification of the strongly acidic cation exchange resin catalyst was conducted in the same method as in Example 1 and modification rate was calculated. The result was shown in Table 2.

(4) Production of Bisphenol A

Using the modified strongly acidic cation exchange resin, bisphenol A was produced in the same method as in Example 1 and results of gas chromatography analysis were shown in Table 2.

Example 16

The strongly acidic cation exchange resin obtained in Example 15 was subjected to elutriation in a range of 140 to 180 μm to obtain exchange capacity, mean particle diameter, particle diameter, uniformity coefficient, and a content of catalyst beads having particle diameters of 650 μm or less in the obtained strongly acidic cation exchange resin, as in Example 1. The results were shown in Table 2.
Modification ofthe strongly acidic cation exchange resin catalyst was conducted in the same method as in Example 1 and modification rate was calculated. The result was shown in Table 2.
Using the modified strongly acidic cation exchange resin, bisphenol A was produced in the same method as in Example 1. Results of gas chromatography analysis were shown in Table 2.

Example 17

The strongly acidic cation exchange resin obtained in Example 15 was subjected to elutriation in a range of 40 to 75 μm As in Example 1, there were obtained exchange capacity, mean particle diameter, uniformity coefficient, and a content of catalyst beads having particle diameters of 650 μm or less in the obtained strongly acidic cation exchange resin, and the results were shown in Table 2.
Modification ofthe strongly acidic cation exchange resin catalyst was conducted in the same method as in Example 1 and modification rate was calculated. The result was shown in Table 2.
Using the modified strongly acidic cation exchange resin, bisphenol A was produced in the same method as in Example 1. Results of gas chromatography analysis were shown in Table 2.
[Table 2]

| | Ex.9 | Ex.10 | Ex.11 | Ex.12 | Ex.13 | Ex.14 | Ex.15 | Ex.16 | Ex.17 |
|---|---|---|---|---|---|---|---|---|---|
| Mean particle diameter (μm) | 183 | 142 | 63 | 205 | 136 | 79 | 218 | 158 | 51 |
| Uniformity coefficient | 1.44 | 1.39 | 1.86 | 1.17 | 1.27 | 1.37 | 1.16 | 1.21 | 1.58 |
| Degree of crosslinking (%) | 2 | 2 | 2 | 4 | 4 | 4 | 6 | 6 | 6 |
| Exchange capacity (meq/g-wet) | 0.96 | 0.95 | 0.99 | 1.61 | 1.50 | 1.65 | 2.07 | 1.98 | 2.06 |
| Modification rate (X) | 15.6 | 13.3 | 15.6 | 15.0 | 15.1 | 15.1 | 15.1 | 14.9 | 15.4 |
| Content of catalyst beads having particle diameters of 650 μm or less (% by volume) | 100 | 100 | 100 | 100 | 100 | 100 | 100 | 100 | 100 |
| TOF(hr-1) | 12.4 | 12.4 | 12.0 | 8.8 | 8.4 | 8.7 | 6.1 | 7.0 | 7.2 |
| 4,4 bisphenol A yield at 60 minutes after start of reaction (%) | 50.2 | 49.5 | 49.9 | 59.7 | 52.9 | 59.9 | 53.3 | 58.7 | 62.3 |
| 4,4'-isomer/2,4'-isomer ratio at 50% yield of 4,4 bisphenol A | 62 | 63 | 65 | 63 | 65 | 67 | 61 | 64 | 72 |

The results hereinabove showed that, in the catalyst for producing a bisphenol compound according to the present invention, even when the particle diameter of gel catalyst beads was made smaller, the 4,4'-isomer/2,2'-isomer ratio was improved while the 4,4'-bisphenol A yield was maintained, thereby allowing an intended 4,4'-bisphenol A to be efficiently produced. It was also found that as the degree of crosslinking became lower, activity per sulfonic acid group (TOF ($hr^{-1}$) was improved, thereby allowing 4,4'-bisphenol A to be produced more efficiently.

Example 18

A stainless steel column having an inner diameter of 1 cm and an entire length of 44 cm was filled with 7.5 mL of the 4-(2-mercaptoethyl)pyridine-modified strongly acidic cation exchange resin catalyst (hereinafter may be referred to as "catalyst A") prepared in Example 1. Phenol of 60°C was fed at a rate of 26 mL/hr for 24 hours from an upper portion of the reactor filled with the catalyst A to completely substitute water in the catalyst A with phenol. After that, a mixture solution in which a ratio of phenol/acetone (molar ratio) was 11 (4.3% by weight of acetone; 79.4% by weight phenol; 10% by weight of 4,4'-bisphenol A; and 6.3% by weight of other substance) was continuously fed at 73°C and at 26 mL/hr in a down-flow manner from the upper portion ofthe reactor to conduct reaction. The reaction solution was collected from a lower portion ofthe reactor to analyze by gas chromatography under the same conditions as in Example 1. Then, based on analysis values, by the following formulas, acetone conversion rate and 4,4'-bisphenol A selectivity were calculated. Figs. 4 and 5 show the results.

$$\text{Acetone Conversion Rate (\%)} = [\{(\text{amount by mole of acetone in 1 Kg of raw material}) - (\text{amount by mole of acetone in 1 Kg of produced liquid})\} / (\text{amount by mole of acetone in 1 Kg of raw material liquid})] \times 100$$

4,4'-bisphenol A selectivity (%) = [(amount by mole of bisphenol A in 1 Kg of

produced liquid) – (amount by mole of bisphenol A in 1 Kg of raw material liquid)] / [(amount by mole of acetone in 1 Kg of raw material liquid) – (amount by mole of acetone in 1 Kg of produced liquid)] $\times$ 100

Comparative Example 4

Production and analysis ofbisphenol A were conducted in the same manner as in Example 18, except for the use ofthe 4-(2-mercaptoethyl)pyridine-modified strongly acidic cation exchange resin catalyst prepared in Comparative Example 1, as an alternative to the catalyst A.
Figs. 4 and 5 show the results.

Example 19

A stainless steel column having the inner diameter of 1 cm and the entire length of 44 cm was filled with 7.5 mL of the 2-(2-mercaptoethyl)pyridine-modified strongly acidic cation exchange resin catalyst (hereinafter may be referred to as "catalyst B") prepared in Example 8. Phenol of 60°C was fed at 26 mL/hr for 24 hours from the upper portion ofthe reactor filled with the catalyst B to completely substitute water in the catalyst B with phenol. After that, a mixture solution (4.3% by weight of acetone; 95.5% by weight of phenol; and 0.2% by weight of others such as an impurity) in which a ratio of phenol/acetone (molar ratio) was 14 was continuously fed at 73°C and at 26 mL/hr in a down-flow manner from the upper portion ofthe reactor to conduct reaction. The reaction solution was collected from the lower portion of the reactor to analyze by gas chromatography under the same conditions as in Example 18. Similarly, acetone conversion rate and 4,4'-bisphenol A selectivity were calculated. Figs. 6 and 7 show the results.

Comparative Example 5

Production and analysis ofbisphenol A were conducted in the same manner as in Example 7, except for the use of the 2-(2-mercaptoethyl)pyridine-modified strongly acidic cation exchange resin catalyst prepared in Comparative Example 3, as an alternative to the catalyst B. Figs. 6 and 7 show the results.
The results hereinabove showed that, in the catalyst for producing a bisphenol compound according to the present invention, by making the particle diameter of the catalyst beads small, not only the initial acetone conversion rate and 4,4'-bisphenol A selectivity were improved, but also high capabilities of the catalyst were maintained for a long period of time, resulting that catalyst life span was also greatly improved, thereby allowing a bisphenol compound to be produced stably and efficiently for a long period of time. Furthermore, it was also found that when using as a promoter 2-(2-mercaptoethyl)pyridine, there was a great effect on the improvement of catalyst life span, thereby allowing 4,4'-bisphenol A to be produced more efficiently.

DESCRIPTION OF REFERENCE NUMERALS

1: Liquid droplet production apparatus
2: Aqueous medium
3: Liquid droplet production tank
4: Hydrophobic liquid
5: Hydrophobic liquid storage tank
6: Hydrophobic liquid supply pipe
7: Nozzle member
8: Underwater speaker
9: Aqueous medium storage tank
10: Aqueous medium supply pipe
11: Ejection hole
12: Hydrophobic liquid ejection/storage tank
13,14: Supply pump
15: Liquid droplet

16: Polymerization reaction apparatus
17: Polymerization reaction tank
18: Liquid droplet transfer pipe
19: Glass column
20: Strongly acidic cation exchange resin filled layer
21: Glass filter
22: Glass filter
23: Column upper line
24: Column upper valve
25: Column lower valve
26: Column lower line

## Claims

**1.** A catalyst for producing a bisphenol compound, composed of gel catalyst beads which comprises
gel beads obtained by the copolymerization reaction of polymerizable monomers including a styrene-based monomer and a crosslinking monomer, and
strongly acidic group introduced in the gel beads, and wherein
50% or more of the gel catalyst beads have particle diameters of 30 to 650 μm

**2.** The catalyst for producing a bisphenol compound according to claim 1, wherein when the catalyst is used in a fixed-bed flow method, 50% or more of the gel catalyst beads have particle diameters of 300 to 600 μm

**3.** The catalyst for producing a bisphenol compound according to claim 1 or 2, wherein a part of the strongly acidic group ofthe catalyst is modified with mercaptoalkylpyridine or mercaptoalkylpyridine having a protected mercapto group.

**4.** The catalyst for producing a bisphenol compound according to claim 3, wherein the mercaptoalkylpyridine is 4-(2-mercaptoethyl)pyridine or 2-(2-mercaptoethyl)pyridine.

**5.** A method for producing a bisphenol compound comprising reacting a phenol compound with a carbonyl compound in the presence of the catalyst for producing a bisphenol compound according to any one of claims 1 to 4.

FIG. 1

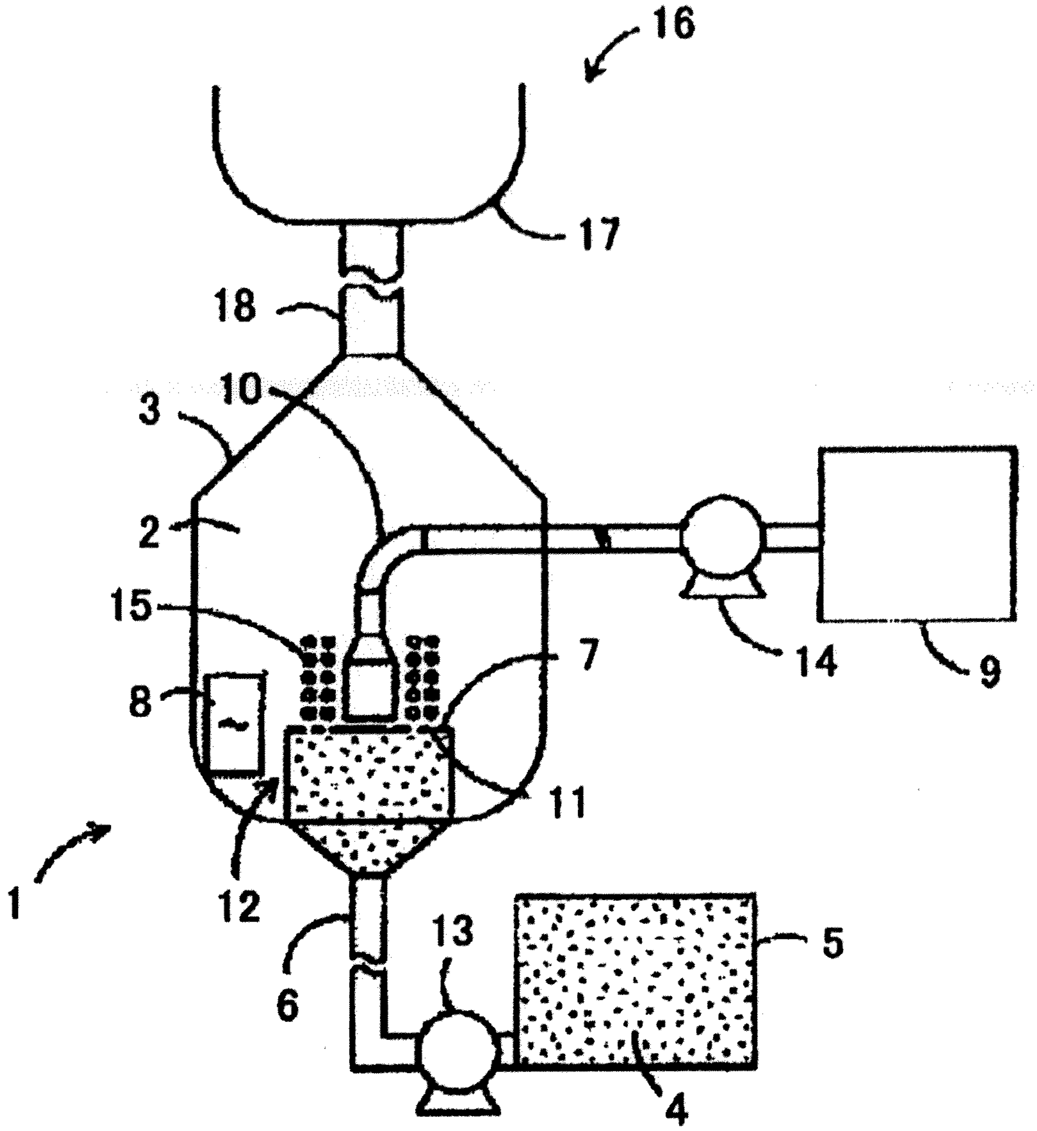

FIG. 2

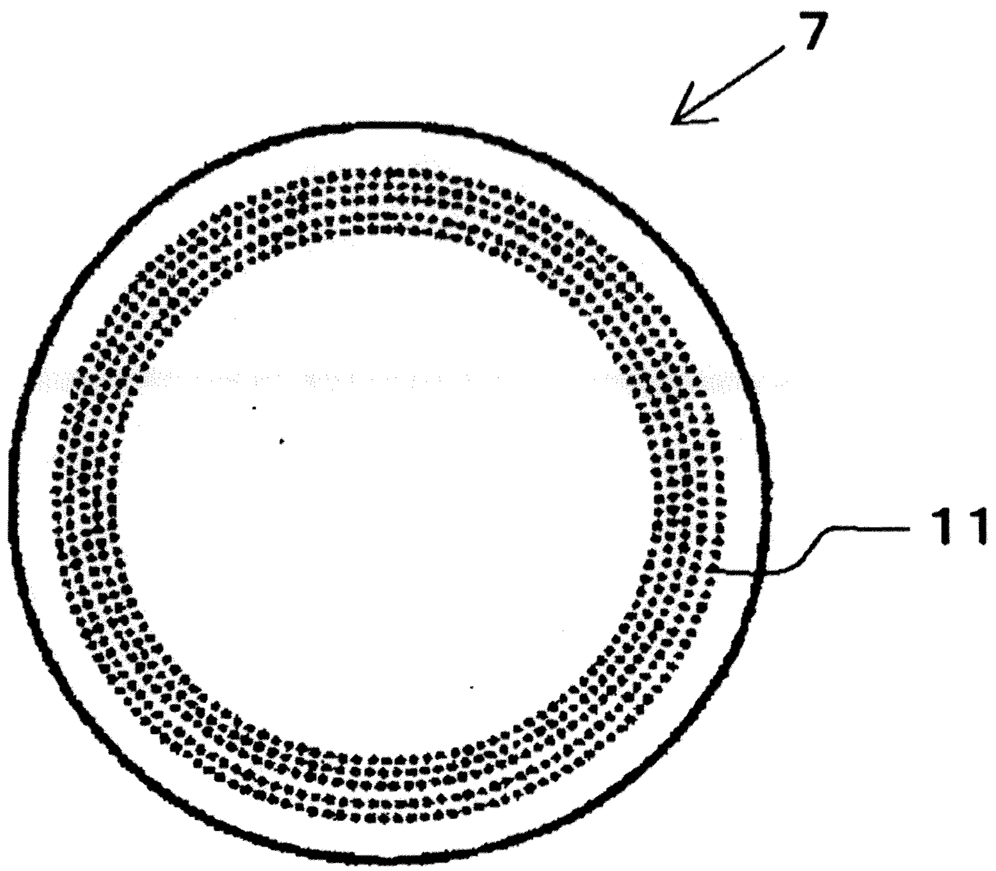

FIG. 3

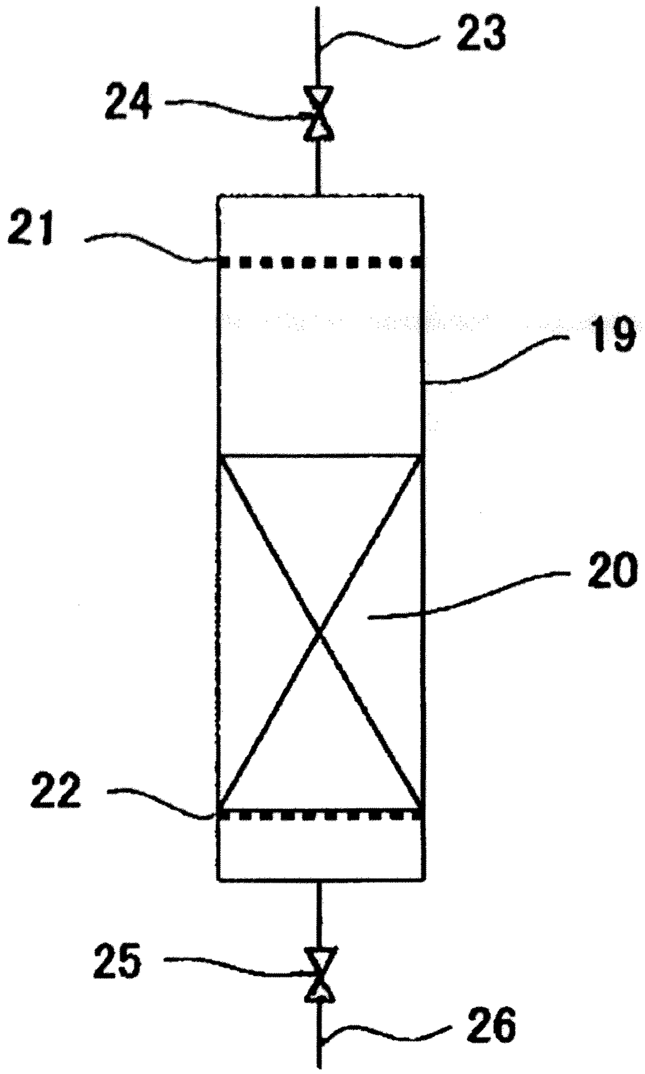

FIG. 4

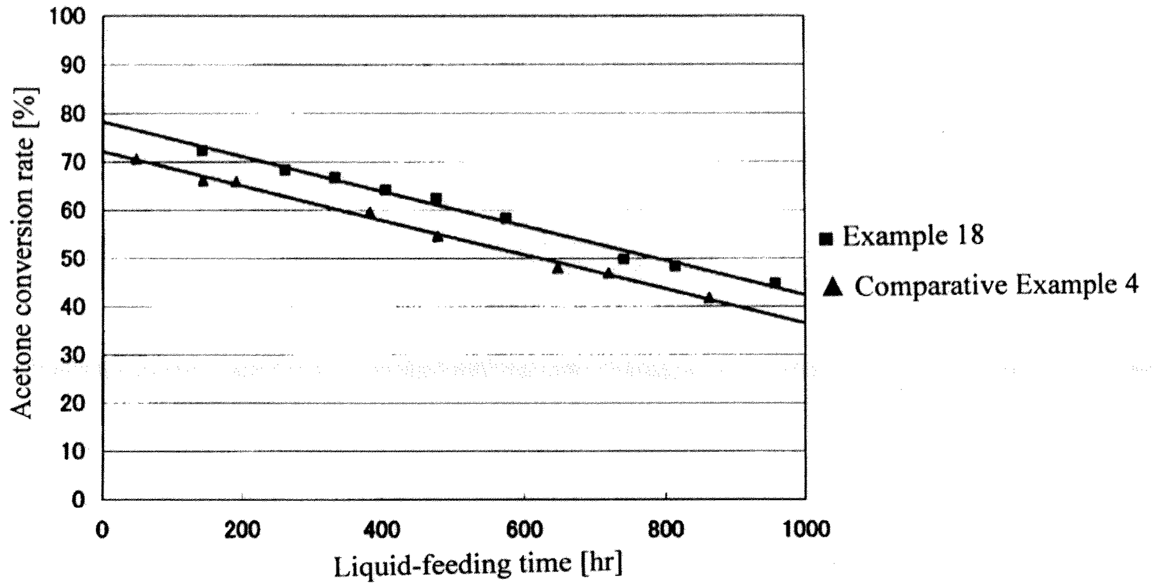
Acetone conversion rate [%]
100
90
80
70
60
50
40
30
20
10
0
0
200
400
600
800
1000
Liquid-feeding time [hr]
Example 18
Comparative Example 4

FIG. 5

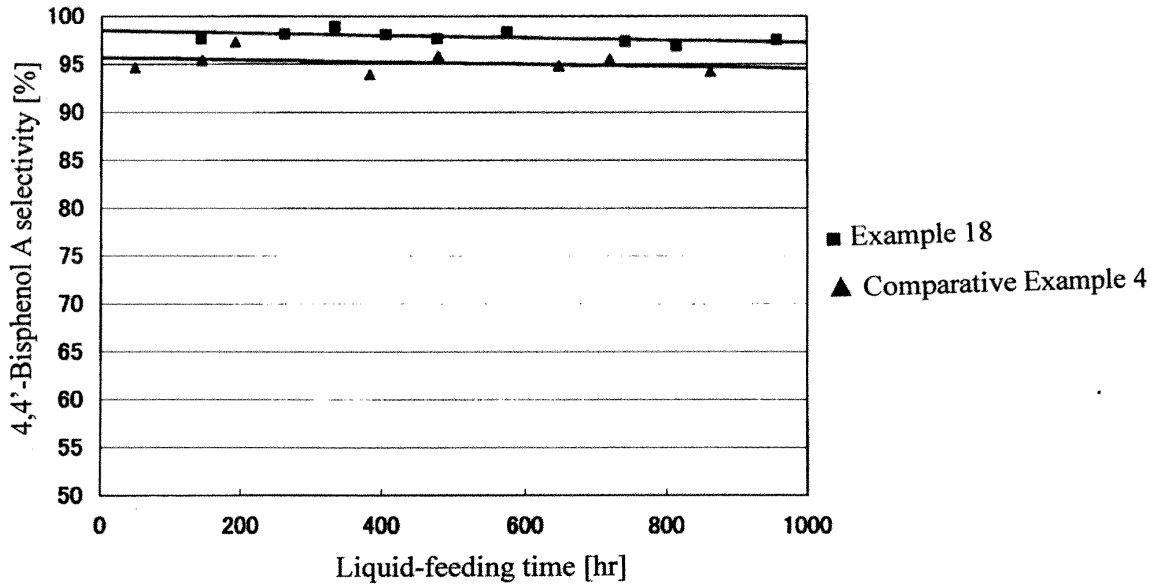
4,4'-Bisphenol A selectivity [%]
100
95
90
85
80
75
70
65
60
55
50
0
200
400
600
800
1000
Liquid-feeding time [hr]
Example 18
Comparative Example 4

FIG. 6

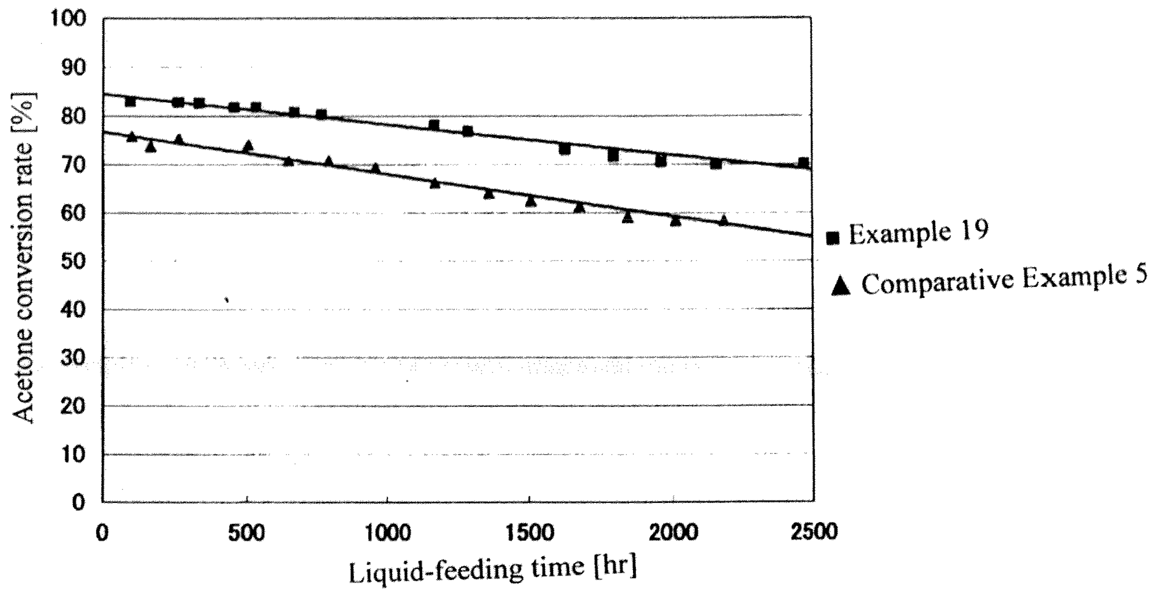

100
90
80
70
60
50
40
30
20
10
0
Acetone conversion rate [%]
0
500
1000
1500
2000
2500
Liquid-feeding time [hr]
■ Example 19
▲ Comparative Example 5


FIG. 7

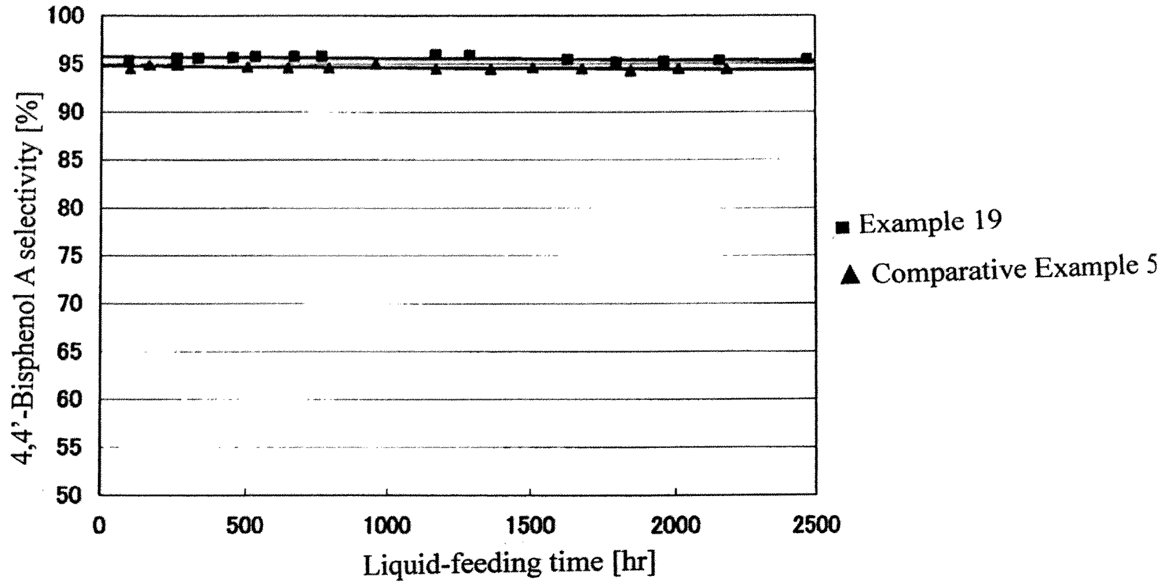

100
95
90
85
80
75
70
65
60
55
50
4,4'-Bisphenol A selectivity [%]
0
500
1000
1500
2000
2500
Liquid-feeding time [hr]
■ Example 19
▲ Comparative Example 5

**INTERNATIONAL SEARCH REPORT**

International application No.
PCT/JP2010/069835

A. CLASSIFICATION OF SUBJECT MATTER
*B01J31/10*(2006.01)i, *C07C37/20*(2006.01)i, *C07C39/16*(2006.01)i, *C07D213/32*(2006.01)i, *C07B61/00*(2006.01)n

According to International Patent Classification (IPC) or to both national classification and IPC

B. FIELDS SEARCHED

Minimum documentation searched (classification system followed by classification symbols)
B01J31/10, C07C37/20, C07C39/16, C07D213/32, C07B61/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | JP 2002-069023 A (Mitsubishi Chemical Corp.), 08 March 2002 (08.03.2002), claims; paragraphs [0006] to [0010]; examples (Family: none) | 1-5 |
| Y | JP 2003-514657 A (Bayer AG.), 22 April 2003 (22.04.2003), paragraph [0021] & US 6723881 B1 & EP 1239958 A & WO 2001/037992 A1 & DE 19956229 A & DE 10027908 A | 1-5 |
| Y | JP 3312920 B2 (Rohm and Haas Co.), 12 August 2002 (12.08.2002), paragraph [0007]; examples & US 5233096 A & EP 486277 A1 | 1-5 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 01 February, 2011 (01.02.11) | 15 February, 2011 (15.02.11) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2010/069835

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2887304 B2 (Chiyoda Corp.), 26 April 1999 (26.04.1999), claims; paragraph [0008]; examples (Family: none) | 1-5 |
| A | JP 2003-252908 A (Mitsubishi Chemical Corp.), 10 September 2003 (10.09.2003), (Family: none) | 1-5 |
| P,X | JP 2009-263309 A (Mitsubishi Chemical Corp.), 12 November 2009 (12.11.2009), claims; paragraphs [0071] to [0081]; examples (Family: none) | 1,2,5 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2001503377 A
- JP 2002205966 A
- JP 2002069023 A
- JP 62178532 A
- JP 3312920 B
- JP 2887304 B
- JP 2003252908 A
- JP 3899786 B


### Non-patent literature cited in the description


- Manual 1 of DIAION: Ion Exchange Resign. Synthetic Absorbent. Mitsubishi Chemical Corp, 31 October 2007, 140-141
- Manual 1 of DIAION: Ion Exchange Resign. Synthetic Absorbent. Mitsubishi Chemical Corp, 31 October 2007, 133-135
- Manual 1 of DIAION: Ion Exchange Resin · Synthetic Absorbent. Mitsubishi Chemical Corp, 31 October 2007, 140-141
- Green's Protective Groups in Organic Synthesis. Wiley, 2007